# EUROPEAN PATENT APPLICATION

(11) **EP 2 433 964 A1**
(43) Date of publication of application: **28.03.2012**
(21) Application number: 10777833.4
(22) Date of filing: 21.05.2010
(51) Int. Cl.: C07K 14/82, A61K 39/00, A61P 35/00, G01N 33/574

(54) **PEPTIDE INDUCING XAGE-1B-SPECIFIC IMMUNE REACTION AND UTILIZATION OF SAME**

(30) Priority: 22.05.2009 JP 2009124315; 15.06.2009 JP 2009142266
(71) Applicant: National University Corporation Okayama University, Kita-ku Okayama-shi Okayama 700-8530 (JP)
(72) Inventor: Nakayama, Eiichi, Okayama-shi Okayama 700-8558 (JP); Ohue, Yoshihiro, Okayama-shi Okayama 700-8558 (JP)
(74) Representative: Bittner, Thomas L.
(86) International application number: PCT/JP2010/058642
(87) International publication number: WO 2010/134601

(57) **Abstract**

The object of the present invention is to clarify functions of XAGE-1b and to develop a vaccine therapy for cancer based on XAGE-1b. Humoral immunity or cellular immunity is induced against XAGE-1b in lung cancer, by use of a peptide comprising an amino acid sequence of any one of (a) through (e) or by use of a peptide comprising an amino acid sequence of any one of (f) through (k).

## Description

### Technical Field

The present invention relates to development of a vaccine therapy of cancer. More specifically, the present invention relates to a peptide which induces humoral immunity and cellular immunity against XAGE-1b in lung cancer, and utilization of the peptide.

### Background Art

The incidence rate of cancer in our country has been increasing year by year, and is currently the most common cause of death. Development of new methods of treatment is urgently required in addition to surgical therapy, chemotherapy, and radiotherapy, each of which is a standard therapeutic method of cancer. Among therapeutic methods of cancer, expectations as a new therapeutic method of cancer are on immunotherapy, which can accurately target cancer cells and which causes few side effects. Particularly, efforts have been made domestically and internationally on development of a vaccine therapy which uses a cancer-specific antigen as its vaccine; the cancer-specific antigen has high immunogenicity with respect to the host immune system.

Out of antigens which have been identified as cancer antigens, the cancer/testis (CT) antigen is expressed in various cancer tissues whereas in normal tissues it is known to be expressed just in the testis. Since the CT antigen is highly cancer-specific, it is considered to be a potential target antigen for cancer vaccines (see for example Non-patent Literatures 1 and 2).

Clinical tests of cancer vaccines targeting the CT antigen have been conducted in many institutes within and outside our country, and some of these tests have demonstrated the usefulness of the cancer vaccines (see for example Non Patent Literatures 3 and 4). For instance, in clinical tests conducted in Okayama University Hospital and Osaka University Hospital to patients suffering from esophagus cancer, prostate cancer, or malignant melanoma, a cancer vaccine which uses NY-ESO-1 protein, i.e. a CT antigen, has been recognized as having a certain effect on reducing and stopping the growth of a tumor (see Non-patent Literature 5).

Until now, the inventors of the present invention identified XAGE-1b, a new CT antigen, by conducting SEREX (serological analysis of cancer antigens by recombinant cDNA expression cloning) method which uses serum of a patient suffering from lung cancer. From a result of performing expression analysis in cancer tissues and normal tissues, XAGE-1b has been found to be specifically expressed in lung cancer, hepatoma, prostate cancer, stomach cancer, and malignant meloma in cases of cancer tissues, and in the testis in cases of normal tissues (see Non-patent Literatures 6 to 10).

Furthermore, it has been reported that the survival term tends to be prolonged in cases where a coexpression of XAGE-1b and HLA class I was observed, and the relation of XAGE-1b with prognosis is gradually getting clarified (see Non-patent Literature 13).

### Citation List

### Non-patent Literature

Non-patent Literature 1
   Boon T, et al. Curr Opin Immunol. 1; 9(5): 681-3. 1997
Non-patent Literature 2
   Scanlan MJ, et al. Immunol Rev. 188; 22-32. 2002
Non-patent Literature 3
   Jager E, et al. Proc Natl Acad Sci U S A. 26; 103(39): 14453-8. 2006
Non-patent Literature 4
   Dutoit V, et al. J Clin Invest 110: 1813-22. 2002
Non-patent Literature 5
   Uenaka A, et al. Cancer Immun. 19; 7: 9. 2007
Non-patent Literature 6
   Ali Eldib AM, et al. Int J Cancer. 10; 108(4): 558-63. 2004
Non-patent Literature 7
   Nakagawa K, et al. Clin Cancer Res. 1; 11(15): 5496-503. 2005
Non-patent Literature 8
   Sato S, et al. Cancer Immun. 5; 7: 5. 2005
Non-patent Literature 9
   Kawabata R, et al. Int J Cancer. 15; 120(10): 2178-84. 2007
Non-patent Literature 10
   Tsuji K, et al. Cancer Immunol Immunother. 57(10): 1429-37. 2008
Non-patent Literature 11
   Scanlan MJ, et al. Cancer Immun. 23; 4:1. 2004
Non-patent Literature 12
   Stockert E, et al. J Exp Med. 20; 187(8): 1349-54. 1998
Non-patent Literature 13
   Kikuchi E. et al. Cancer Immun. 28; 8: 13. 2008

### Summary of Invention

### Technical Problem

Surely, the CT antigen is favorable as a target antigen for a cancer vaccine, and is expected that XAGE-1b, a CT antigen, functions as a target antigen of the cancer vaccine. However, not all CT antigens induce humoral immunity reactions. For instance, although CT antigens such as MAGE and SSX are expressed in various cancer tumors, hardly any humoral immunity reaction has been observed (see Non-patent Literatures 11 and 12). As such, with the CT antigens reported in the past, the frequency that an antibody is present in the serum of patients suffering from cancer is extremely low. Moreover, since XAGE-1b is confirmed as an intranuclear antigen, it is difficult to consider that XAGE-1b would induce humoral immunity in a degree effective for reduction or treatment of cancer.

Moreover, although Non-patent Literature 13 does report that the survival term is prolonged of patients suffering from lung adenocarcinoma in which the coexpression of XAGE-1b and HLA class I molecules were observed, with patients with a decreased expression of HLA class I molecules, no prolonging of the survival term is recognized, even if XAGE-1b were expressed. Therefore, just because it was observed in some lung adenocarcinoma patients that prognosis was good in cases where the XAGE-1b and the HLA class I molecules were coexpressed, it is difficult to consider that XAGE-1b is effective for reducing and treating cancer of all types which express XAGE-1b.

The present invention is accomplished in view of the foregoing problems, and its object is to clarify functions of XAGE-1b and to develop a cancer vaccine therapy based on XAGE-1b.

### Solution to Problem

The present invention is accomplished in order to attain the object of the invention as a result of originality and ingenuity of the inventors of the present invention. Namely, the present invention provides a specific fragment of XAGE-1b, and utilization thereof.

The present invention provides a peptide useful for diagnosing lung cancer, and a composition containing the peptide. The peptide is made up of an amino acid sequence of any one of the following (a) through (e):
(a) an amino acid sequence from position-1 to position-25 of SEQ ID. No. 1;
(b) an amino acid sequence from position-15 to position-53 of SEQ ID. No. 1;
(c) an amino acid sequence being a partial sequence of the amino acid sequence from position-15 to position-53 of SEQ ID. No. 1, the partial sequence including an amino acid sequence from position-15 to position-39, from position-29 to position-53, from position-21 to position-48, from position-21 to position-36, from position-25 to position-40, from position-29 to position-44, or from position-33 to position-48, each of SEQ ID. No. 1;
(d) an amino acid sequence from position-43 to position-81 of SEQ ID. No. 1; and
(e) an amino acid sequence being a partial sequence of the amino acid sequence from position-43 to position-81 of SEQ ID. No. 1, the partial sequence including an amino acid sequence from position-43 to position-67, from position-57 to position-81, from position-57 to position-72, or from position-65 to position-81, each of SEQ ID. No. 1. The lung cancer may be non-small-cell lung cancer or lung adenocarcinoma.

The present invention further provides a method of diagnosing lung cancer with use of the peptide or an antibody against the peptide. The method of diagnosing lung cancer includes measuring a level of peptide present in a sample derived from a subject, or measuring a level of an antibody binding specifically to the peptide.

The peptide is useful for inducing humoral immunity against lung cancer. Namely, the present invention provides a composition for inducing humoral immunity against lung cancer, which composition contains the peptide.

The present invention further provides a peptide useful for inducing cellular immunity against lung cancer, and a composition containing the peptide. The peptide is made up of an amino acid sequence of any one of the following (f) through (k):
(f) an amino acid sequence from position-1 to position-39 of SEQ ID. No. 1;
(g) an amino acid sequence being a partial sequence of the amino acid sequence from position-1 to position-39 of SEQ ID. No. 1, the partial sequence including an amino acid sequence from position-1 to position-25, from position-15 to position-39, from position-13 to position-36, from position-13 to position-28, from position-17 to position-32, from position-21 to position-36, from position-9 to position-24, or from position-21 to position-29, each of SEQ ID. No. 1;
(h) an amino acid sequence from position-29 to position-53 of SEQ ID. No. 1;
(i) an amino acid sequence being a partial sequence of the amino acid sequence from position-29 to position-53 of SEQ ID. No. 1, the partial sequence including an amino acid sequence from position-29 to position-48, from position-29 to position-44, or from position-33 to position-48, each of SEQ ID. No. 1;
(j) an amino acid sequence from position-43 to position-81 of SEQ ID. No. 1; and
(k) an amino acid sequence being a partial sequence of the amino acid sequence from position-43 to position-81 of SEQ ID. No. 1, the partial sequence including an amino acid sequence from position-43 to position-67, from position-57 to position-81, from position-53 to position-68, from position-49 to position-64, from position-50 to position-60, or from position-51 to position-59, each of SEQ ID. No. 1. The lung cancer may be non-small-cell lung cancer or lung adenocarcinoma.

For a fuller understanding of the nature and advantages of the invention, reference should be made to the ensuing detailed description taken in conjunction with the accompanying drawings.

### Advantageous Effects of Invention

The present invention thus allows for examination or diagnosis of cancer, prevention of cancer or treatment of cancer.

### Brief Description of Drawings

Fig. 1

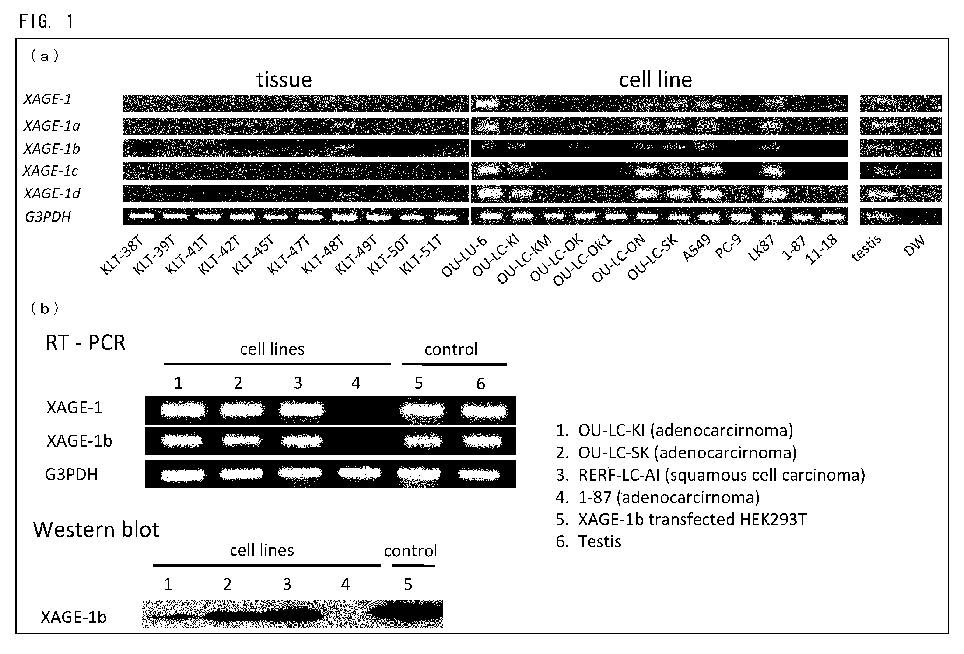

   Fig. 1 is a view showing a result of a RT-PCR analysis of XAGE-1 in non-small-cell lung cancer; (a) shows how expression of XAGE-1b differs between cell lines and tissues in non-small-cell lung cancer, and (b) shows that XAGE-1b protein is also present in the cell lines.
Fig. 2

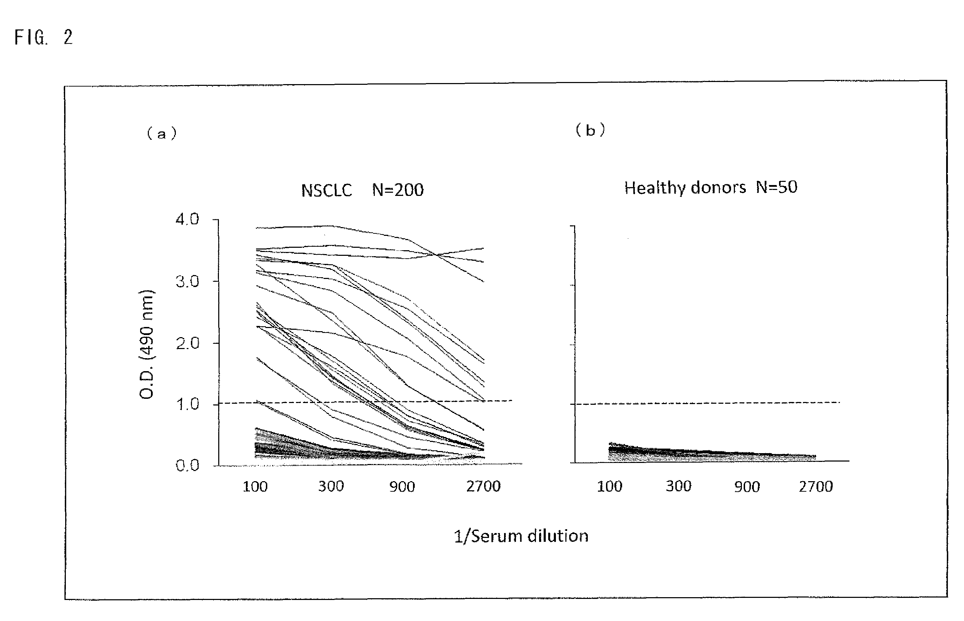

   Fig. 2 is a view showing antibody responses to XAGE-1b protein in patients of non-small-cell lung cancer; (a) shows absorbance (OD value) measured by ELISA of non-small-cell lung cancer patients and (b) shows that of healthy donors.
Fig. 3

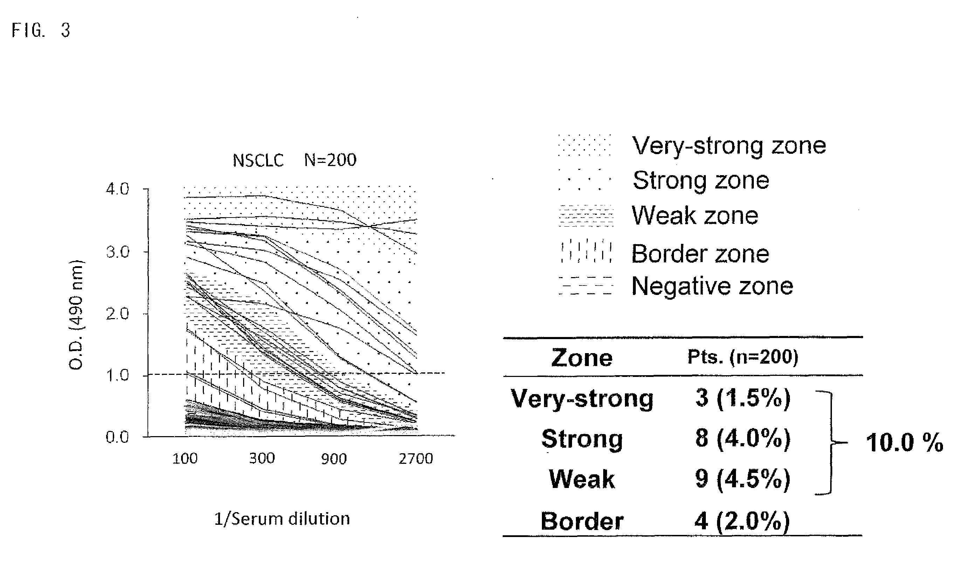

   Fig. 3 is a view showing zones of antibody responses to XAGE-1b protein in patients suffering from non-small-cell lung cancer.
Fig. 4

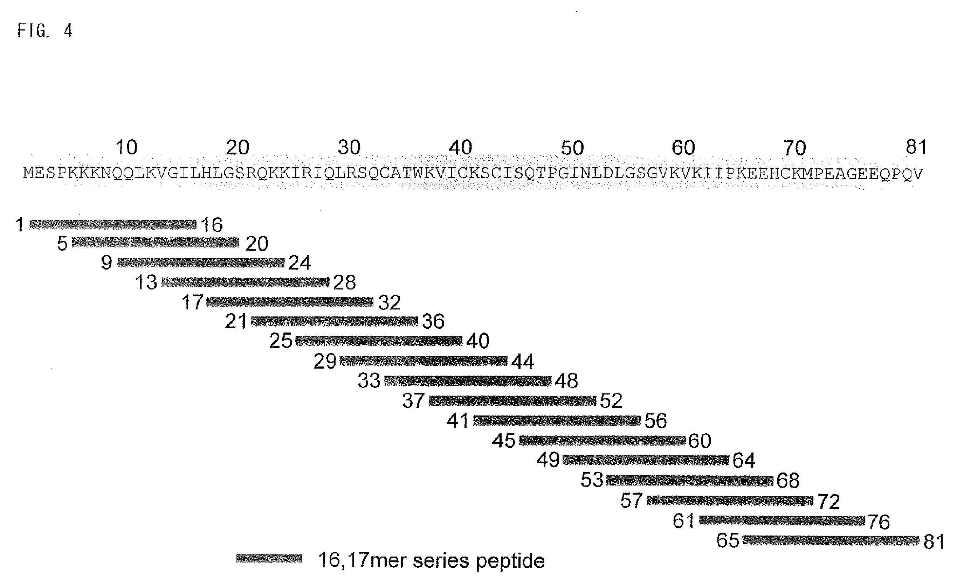

   Fig. 4 is a view showing an amino acid sequence of XAGE-1b overlapping peptides which are used in epitope analysis.
Fig. 5

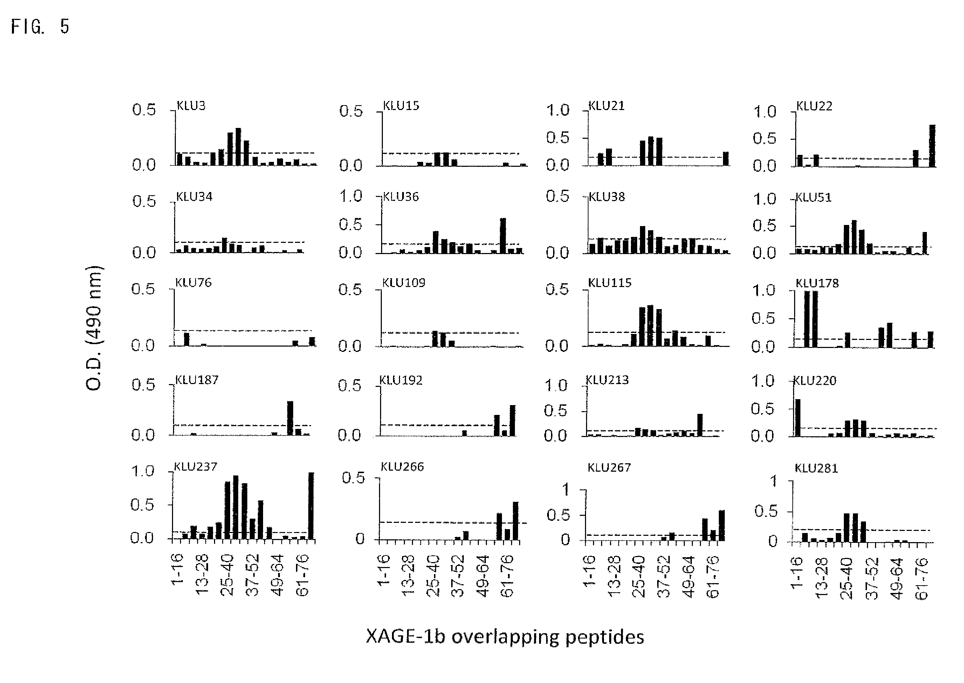

   Fig. 5 is a view showing antibody recognition with respect to XAGE-1b overlapping peptides.
Fig. 6

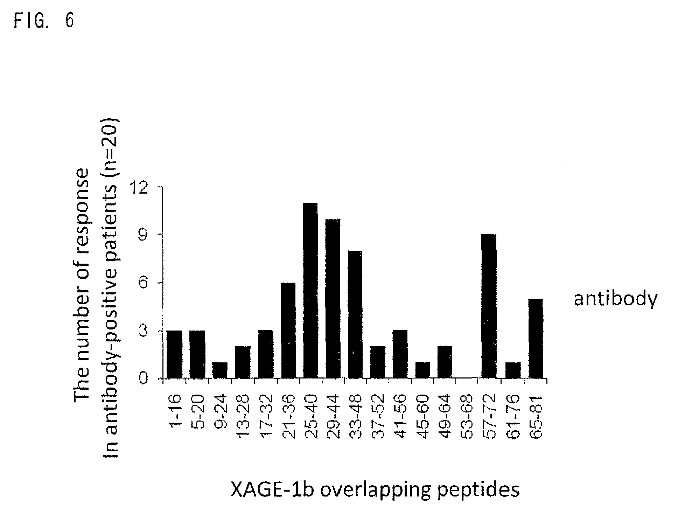

   Fig. 6 is a view showing zones of XAGE-1b that are recognized by anti-XAGE-1b antibodies, in serum antibody-positive patients (n = 20).
Fig. 7

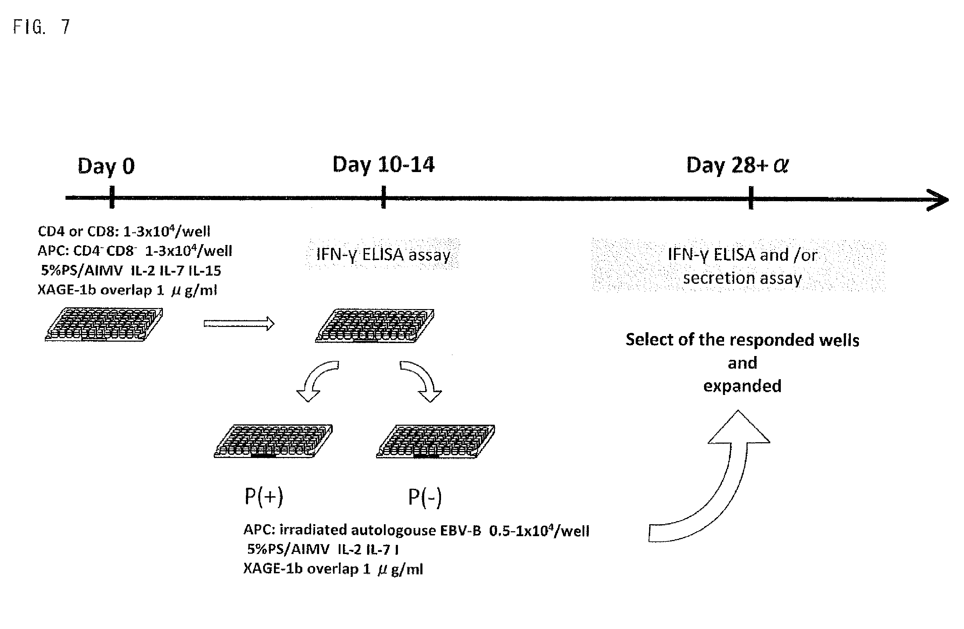

   Fig. 7 is a view showing a procedure for inducing specific T-cells.
Fig. 8

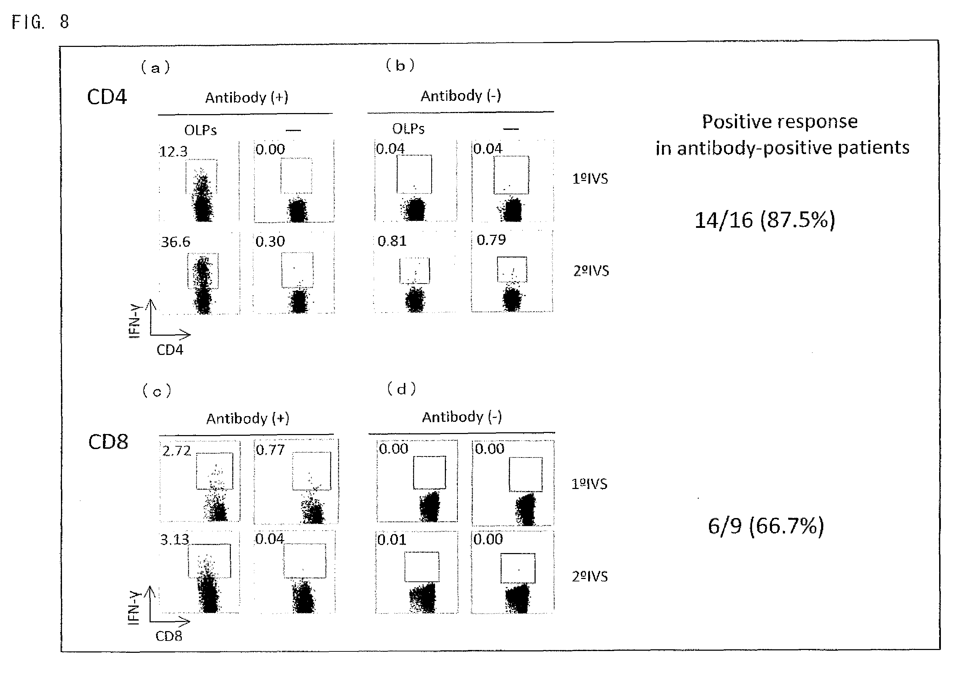

   Fig. 8 is a view showing that XAGE-1b-specific T-cells are induced in serum antibody-positive patients; (a) shows that antigen-specific CD4 positive T-cells were successively induced in the serum antibody-positive patients and (c) shows that antigen-specific CD8-positive T-cells were successively induced in the serum antibody-positive patients, whereas (b) and (d) illustrate that no antigen-specific reaction could be observed in five serum antibody-negative patients or five healthy persons.
Fig. 9

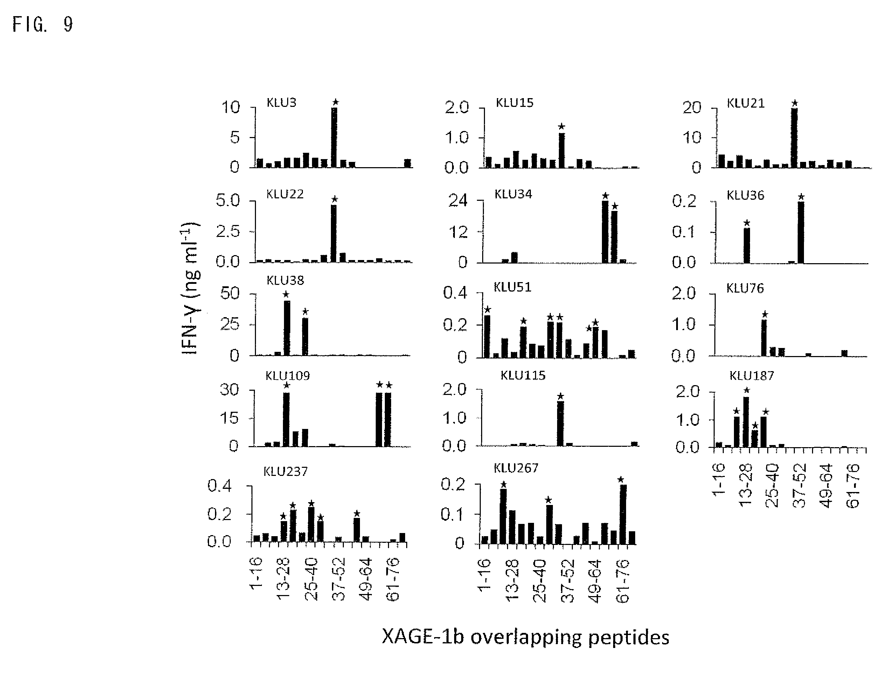

   Fig. 9 is a view showing zones of XAGE-1b that are recognized by specific CD4-positive T-cells (n = 14).
Fig. 10

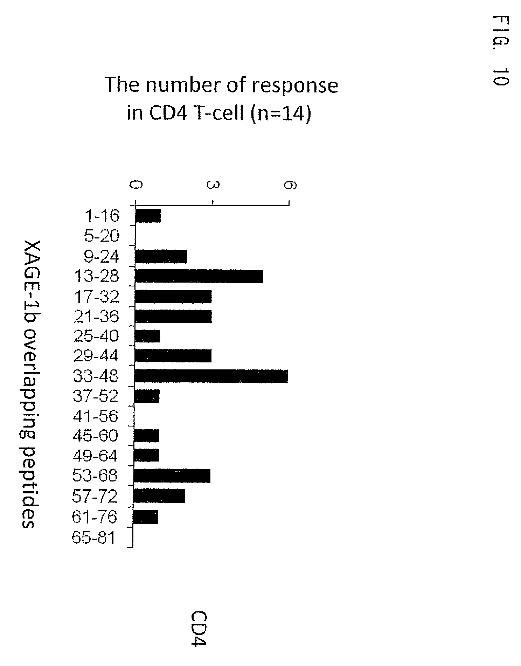

   Fig. 10 is a view showing main epitope zones of XAGE-1b overlapping peptides which are recognized by XAGE-1b-specific CD4-positive T-cells (n = 14).
Fig. 11

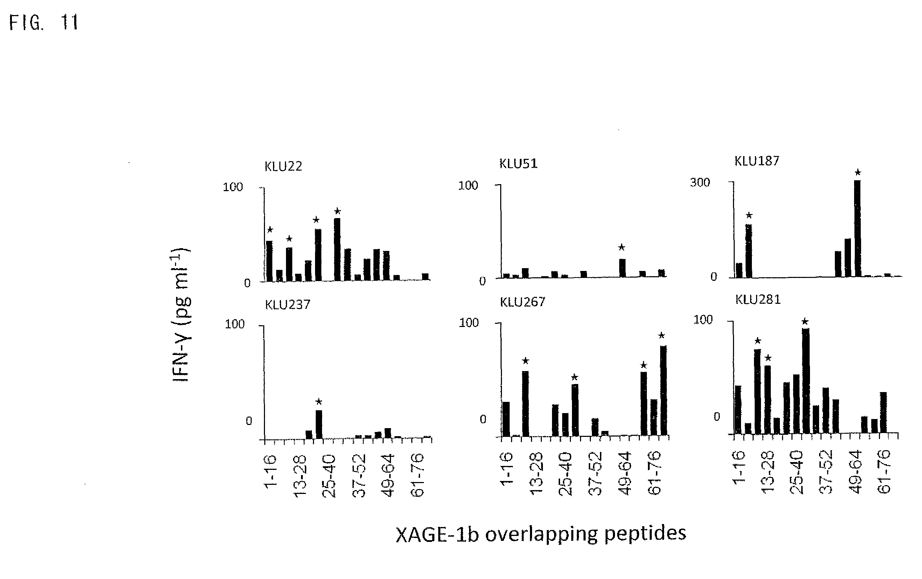

   Fig. 11 is a view showing zones of XAGE-1b that are recognized by specific CD8-positive T-cells (n = 6).
Fig. 12

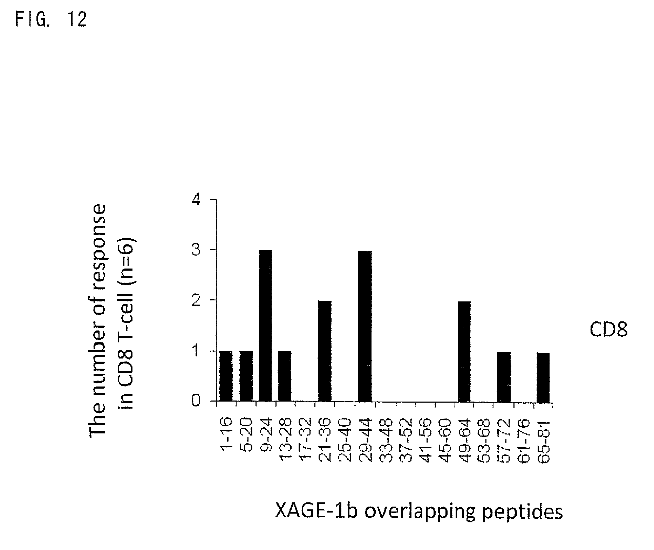

   Fig. 12 is a view showing main epitope zones that are recognized by XAGE-1b-specific CD8-positive T-cells (n = 6).
Fig. 13

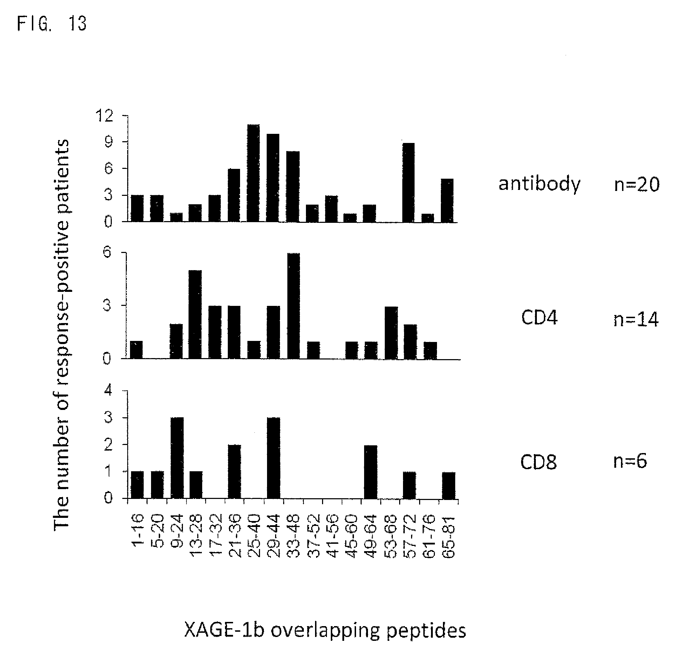

   Fig. 13 is a view showing main epitope zones that are recognized by a XAGE-1b-specific antibody, XAGE-1b-specific CD4-positive T-cells, or XAGE-1b-specific CD8-positive T-cells.
Fig. 14

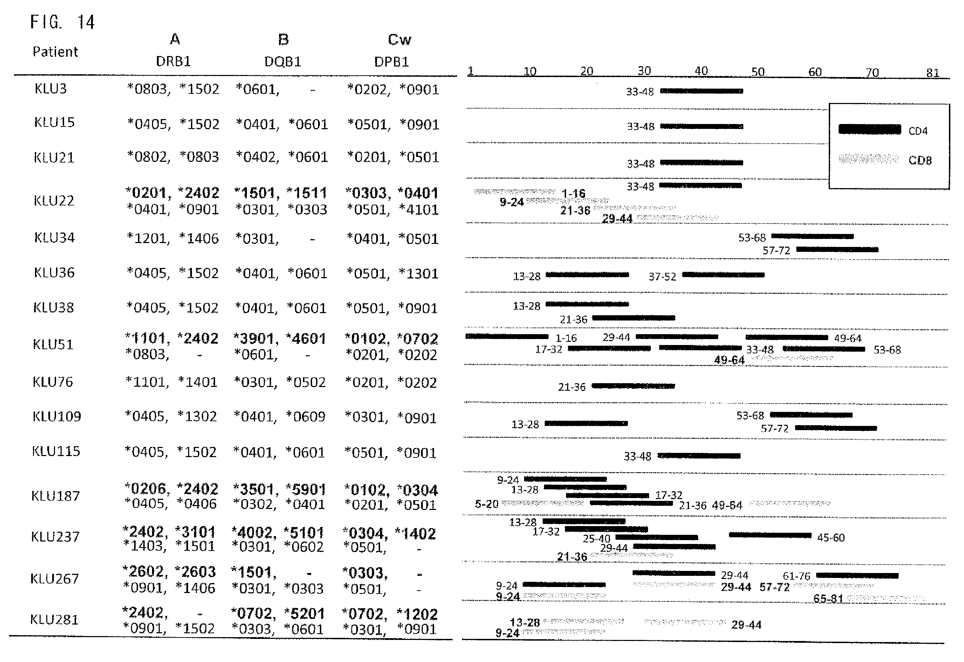

   Fig. 14 shows a relation of main epitope zones with HLA, which main epitope zones are recognized by XAGE-1b-specific CD4-positive T-cells and CD8-positive T-cells.
Fig. 15

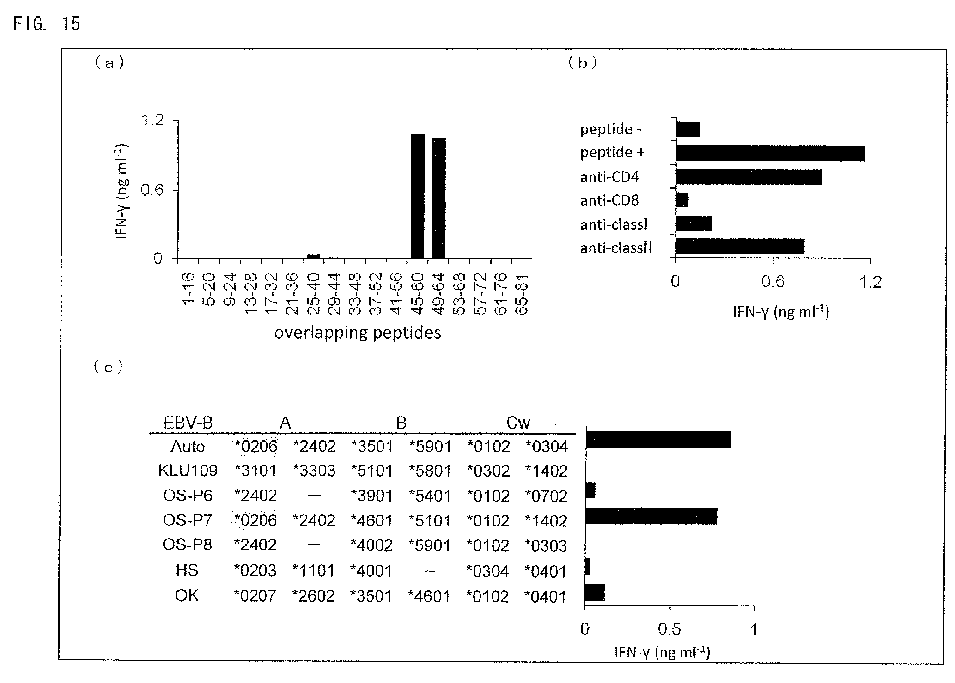

   Fig. 15 is a view showing properties of clone T cells (8C187-1) established from case KLU187; (a) shows that the established T-cells recognize an amino acid sequence from position-45 to position-64 of the amino acid sequence of XAGE-1b, (b) shows that the T-cells are HLA class I-restricted and CD8-restricted, and (c) shows that the CD8-positive T-cells (8C187-1) are HLA-A*0206-restricted.
Fig. 16

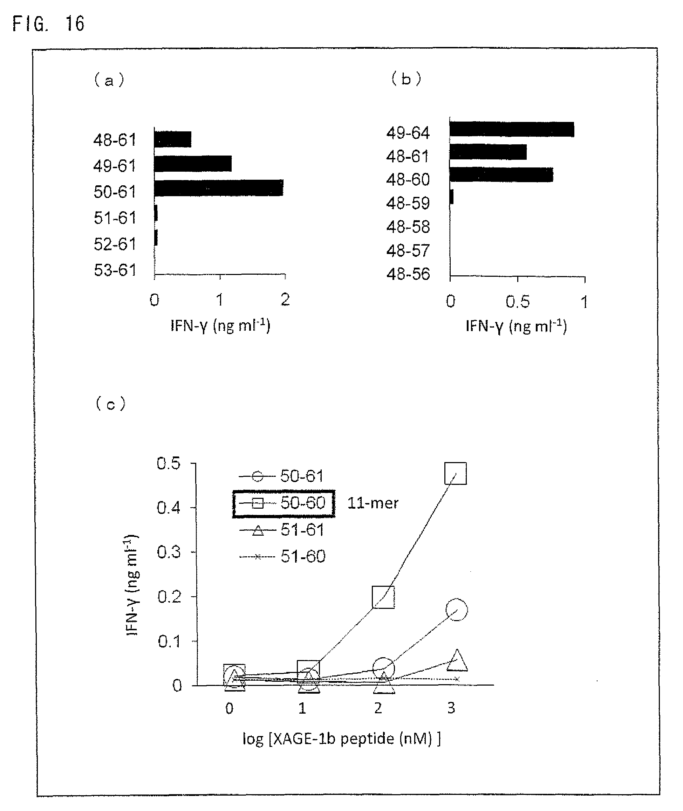

   Fig. 16 is a view showing that a minimal peptide zone of XAGE-1b recognized by HLA-A*0206-restricted CD8-positive T-cells is from position-50 to position-60 of the amino acid sequence of XAGE-1b; (a) and (b) show a result of examining a minimal epitope recognized by the CD8-positive T-cells, and (c) shows that the minimal epitope zone is from position-50 to position-60 of the amino acid sequence of XAGE-1b.
Fig. 17

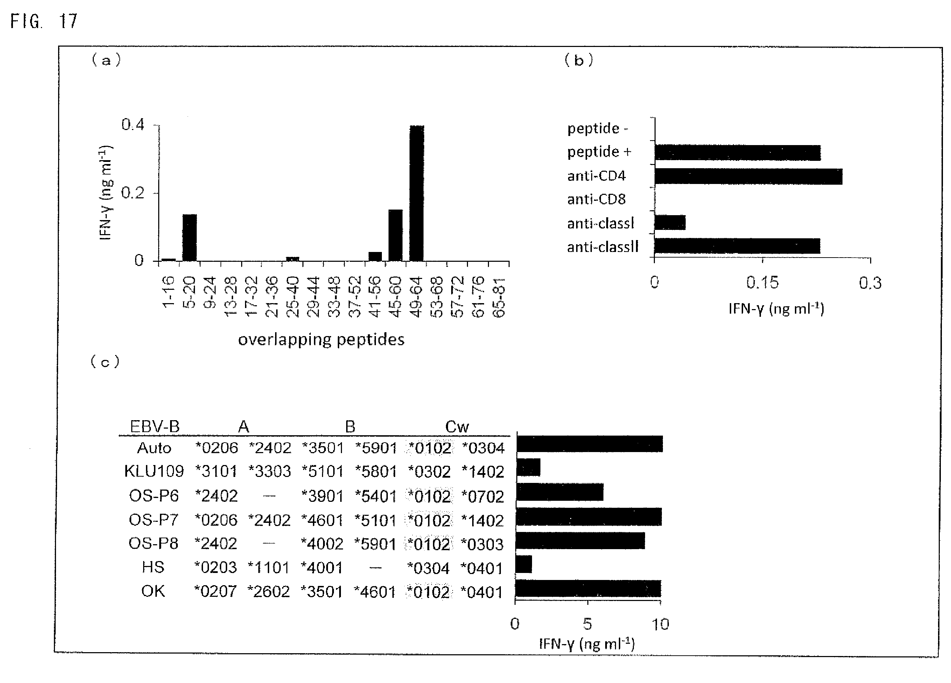

   Fig. 17 is a view showing properties of clone T-cells (8C187-2) established from case KLU187; (a) shows that the established T-cells recognize an amino acid sequence from position-49 to position-64 of the amino acid sequence of XAGE-1b, (b) shows that the T-cells are HLA class I-restricted and CD8-restricted, and (c) shows that the CD8-positive T-cells (8C187-2) are HLA-Cw*0102-restricted.
Fig. 18

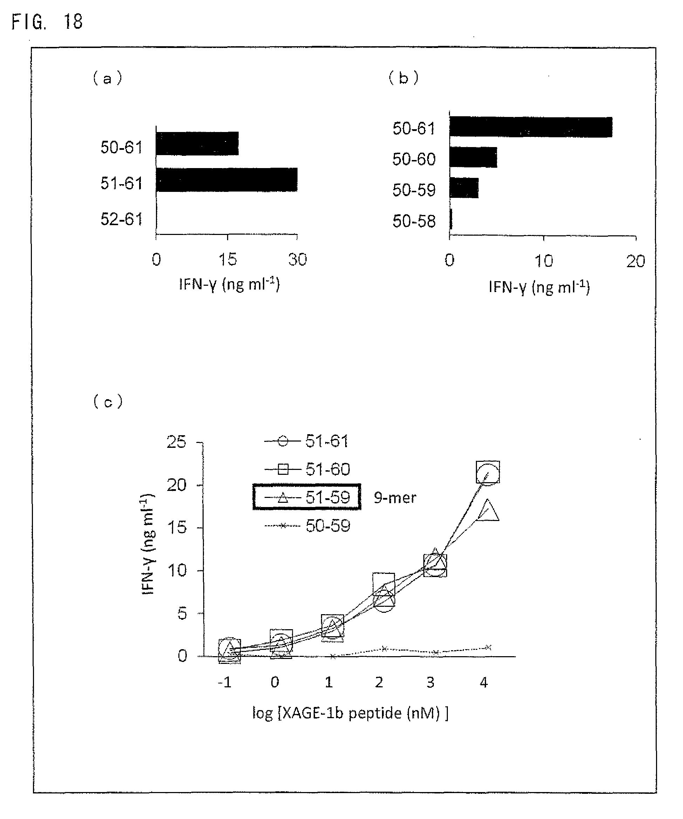

   Fig. 18 is a view showing that a minimal peptide zone of XAGE-1b recognized by HLA-Cw*0102-restricted CD8-positive T-cells is from position-51 to position-59 of the amino acid sequence of XAGE-1b; (a) and (b) show a result of examining a minimal epitope recognized by the CD8-positive T-cells, and (c) shows that the epitope zone is from position-51 to position-59 of the amino acid sequence of XAGE-1b.
Fig. 19

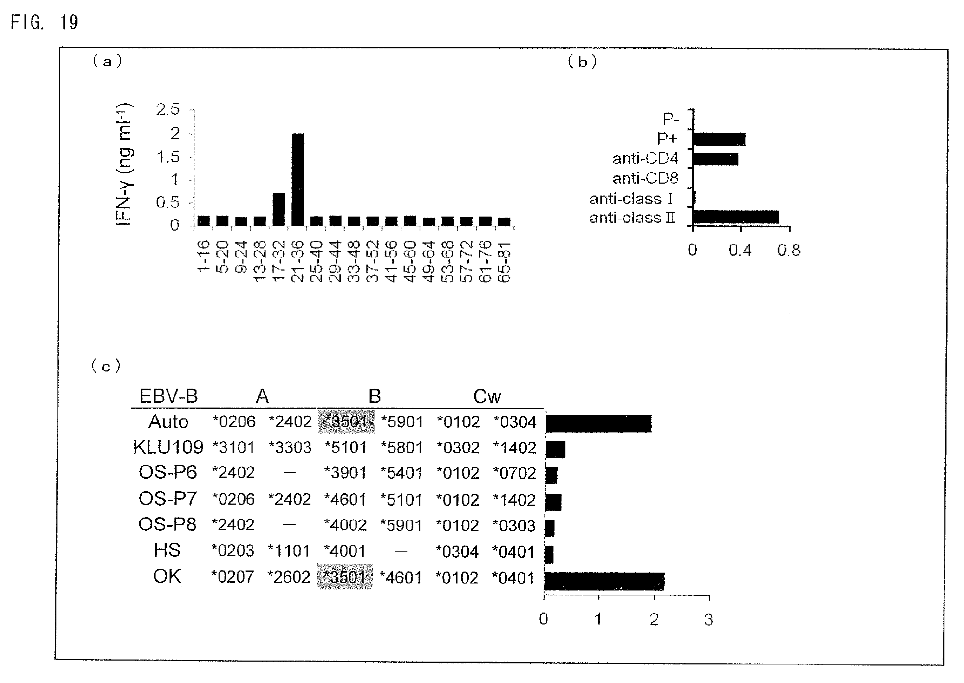

   Fig. 19 is a view showing properties of clone T-cells (8C187-4) established from case KLU187; (a) shows that the established T-cells recognize an amino acid sequence from position-21 to position-36 of the amino acid sequence of XAGE-1b, (b) shows that the T-cells are HLA class I-restricted and CD8-restricted, and (c) shows that the CD8-positive T-cells (8C187-4) are HLA-B*3501-restricted.
Fig. 20

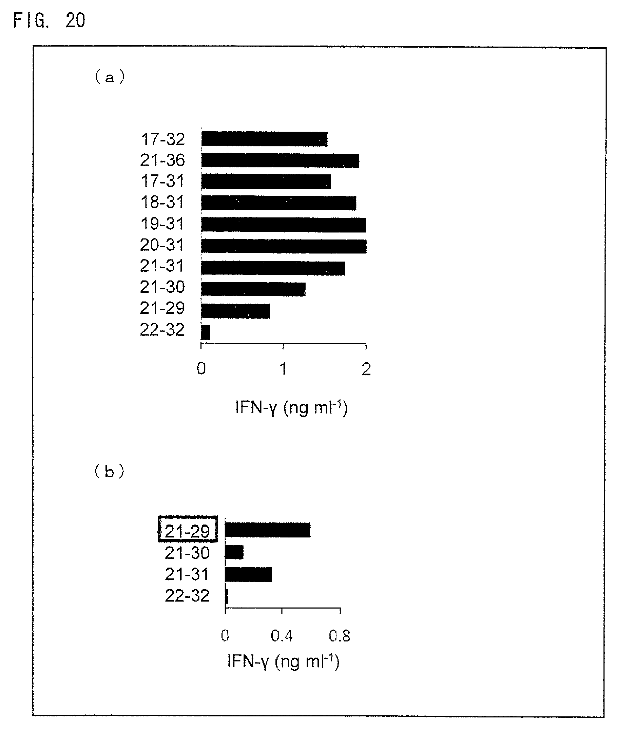

   Fig. 20 is a view showing that a minimal peptide zone of XAGE-1b that is recognized by HLA-B*3501-restricted CD8-positive T-cells is from position-21 to position-29 of the amino acid sequence of XAGE-1b; (a) shows a result of examining a minimal epitope recognized by the CD8-positive T-cells, and (b) shows that an epitope zone is from position-21 to position-29 of the amino acid sequence of XAGE-1b.
Fig. 21

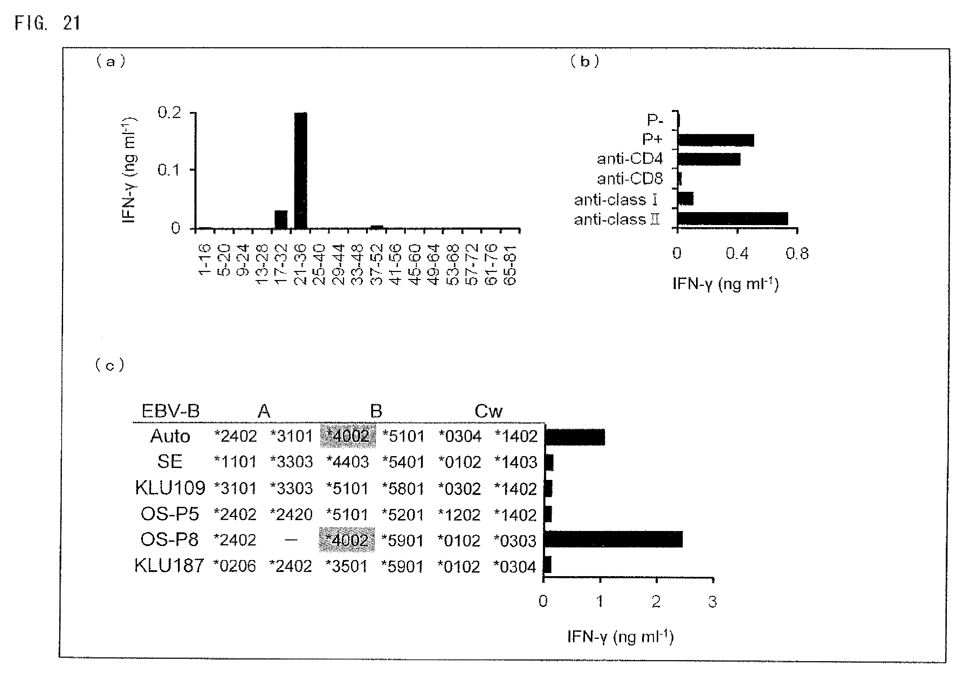

   Fig. 21 is a view showing properties of clone T-cells (8C237-22) established from case KLU237; (a) shows that the established T-cells recognize an amino acid sequence from position-21 to position-36 of the amino acid sequence of XAGE-1b, (b) shows that the T-cells are HLA class I-restricted and CD8-restricted, and (c) illustrates that the CD8-positive T-cells (8C237-22) are HLA-B*4002-restricted.
Fig. 22

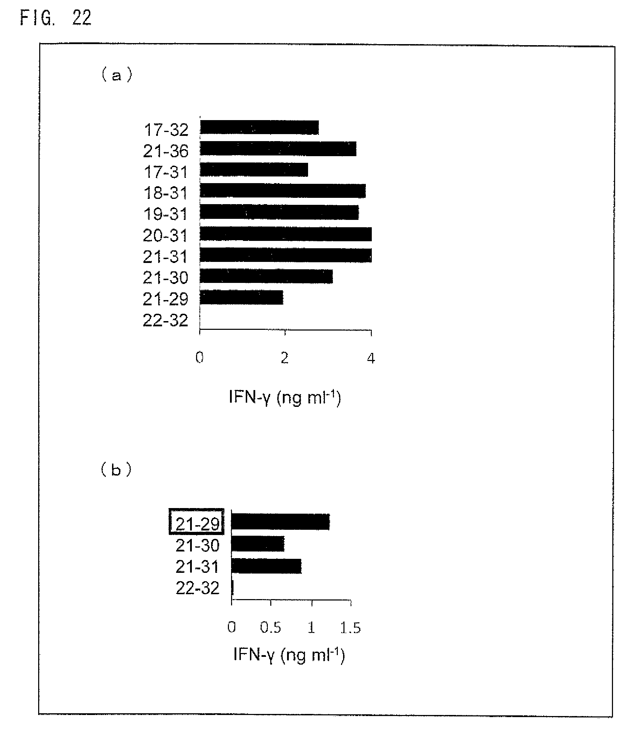

   Fig. 22 is a view showing that a minimal peptide zone of XAGE-1b that is recognized by the HLA-B*4002-restricted CD8 T-cell is from position-21 to position-29 of the amino acid sequence of XAGE-1b; (a) shows a result of examining a minimal epitope that is recognized by the CD8-positive T-cells, and (b) shows that the epitope zone is from position-21 to position-29 of the amino acid sequence of XAGE-1b.
Fig. 23

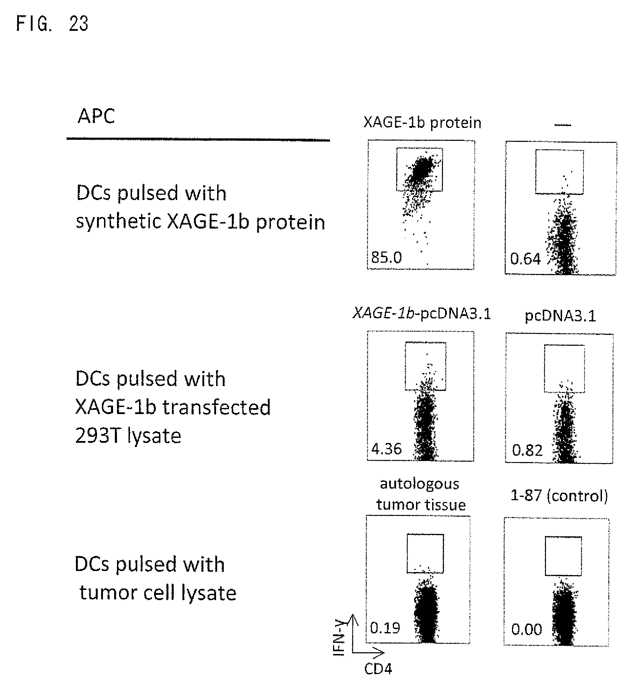

   Fig. 23 is a view showing that the obtained CD4 clone T-cells (4C187-1) recognize a natural epitope.
Fig. 24

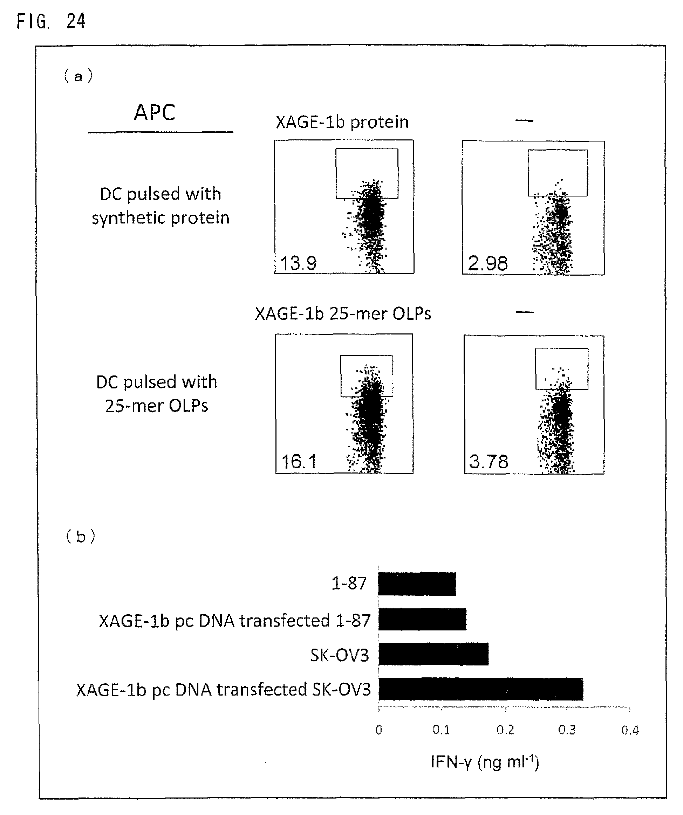

   Fig. 24 is a view showing that the obtained CD8 clone T-cells (8C187-4) recognize a natural epitope; (a) shows that the 8C187-4 recognize XAGE-1b protein and 25-mer XAGE-1b overlapping peptides, and (b) shows that the 8C187-4 specifically recognize a B*3501-positive tumor cell in which plasmid DNA of XAGE-1b is transfected.
Fig. 25

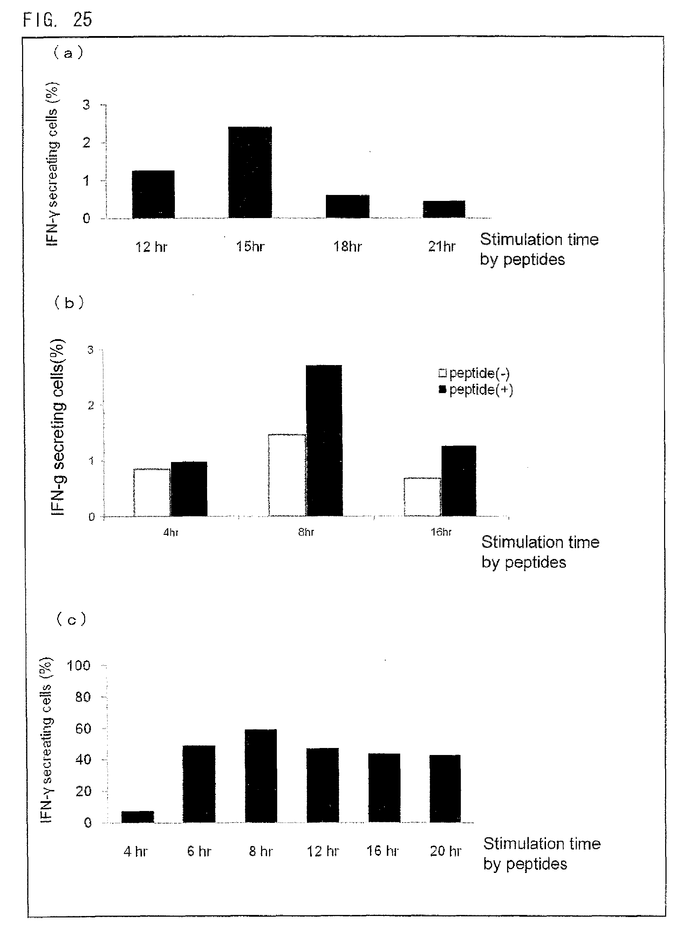

   Fig. 25 is a view showing induction of a specific immune reaction against XAGE-1b in accordance with reaction time of antigen with cells; (a) is a view showing a result obtained in the presence of IL-2 and IL-7, and (b) and (c) are views showing results obtained in the presence of IL-7 and IL-15.
Fig. 26

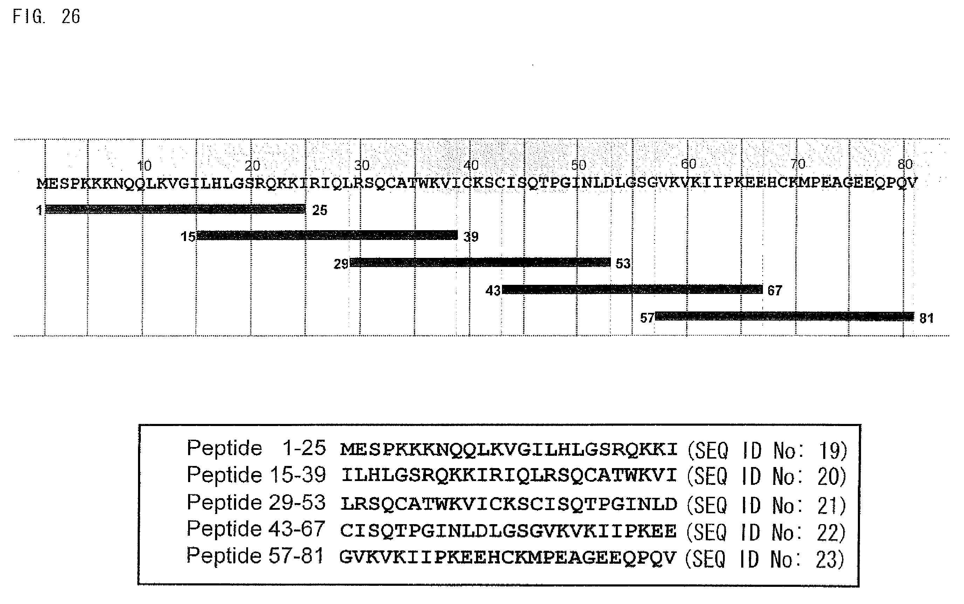

   Fig. 26 is a view illustrating an amino acid sequence of a long chain peptide of XAGE-1b.
Fig. 27

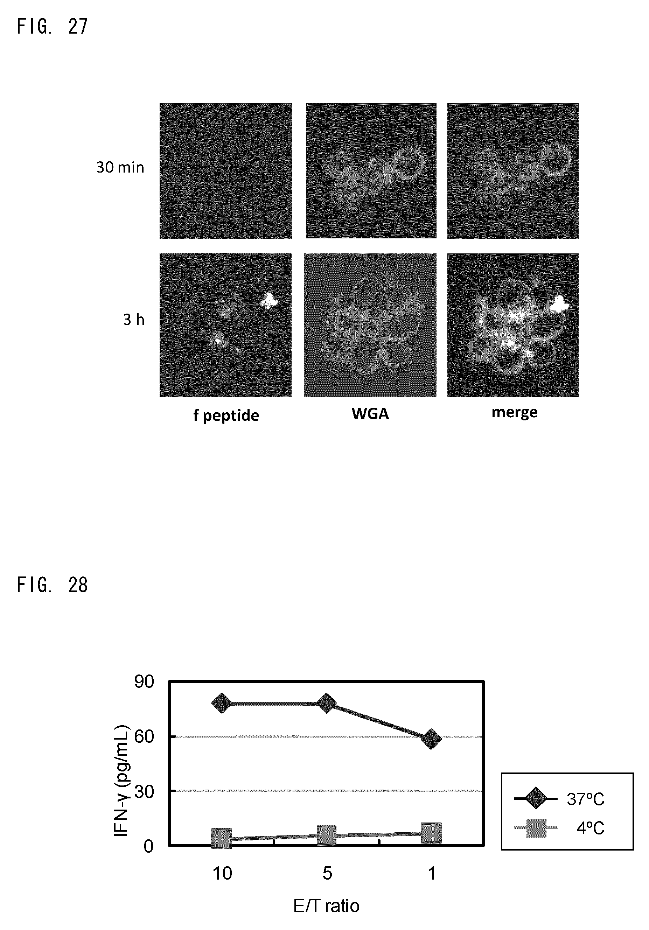

   Fig. 27 is a view showing that NY-ESO-1 f peptide is taken into an antigen-presenting cell (U937).
Fig. 28
   Fig. 28 is a view showing that NY-ESO-1 f peptide is presented as an antigen by MHC class II.
Fig. 29

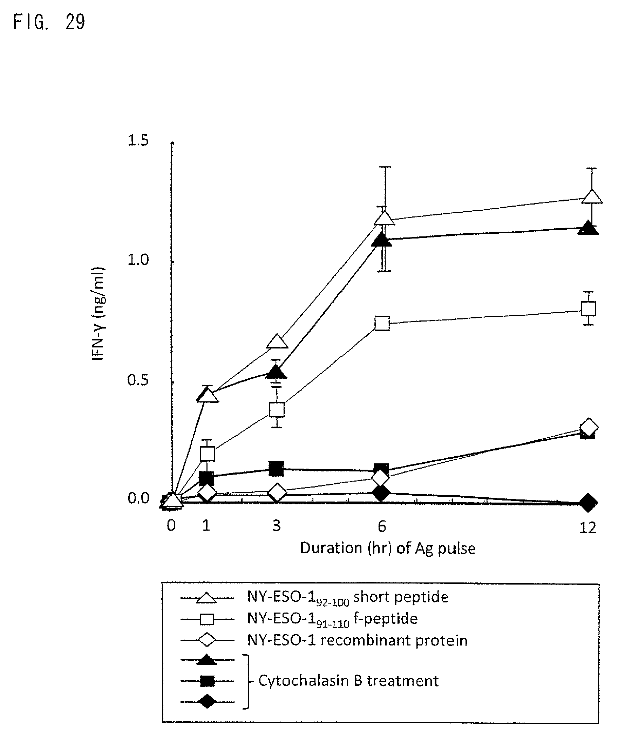

   Fig. 29 is a view illustrating that NY-ESO-1 f peptide is presented as an antigen by MHC class I.
Fig. 30

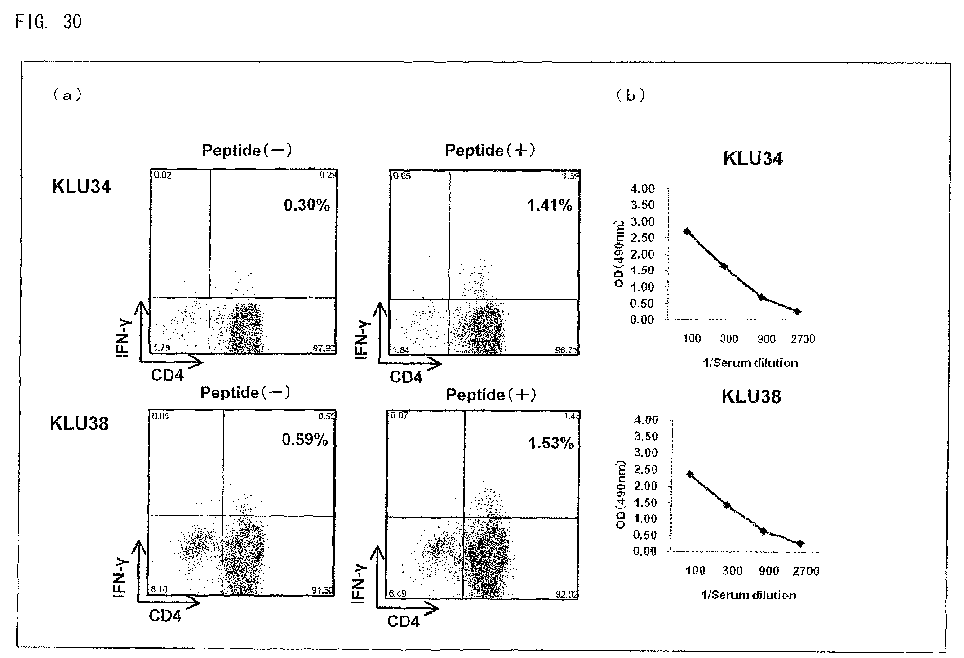

   Fig. 30 is a view showing responses of CD4-positive T-cells to a XAGE-1b peptide upon stimulation and culturing twice, in two typical cases (KLU34 and KLU38) where reaction was observed; (a) shows a result of flow cytometry, and (b) shows a result of measuring serum antibody titer of patients by ELISA.
Fig. 31

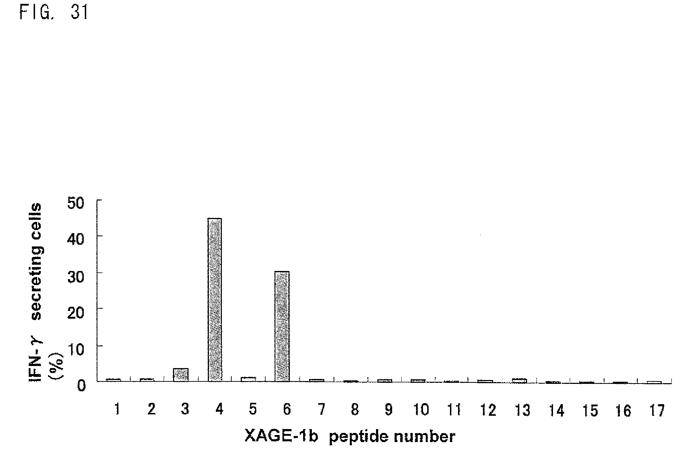

   Fig. 31 is a view showing zones of XAGE-1b recognized by XAGE-1b-specific CD4-positive T-cells in a serum antibody titer positive-patient (KLU38).
Fig. 32

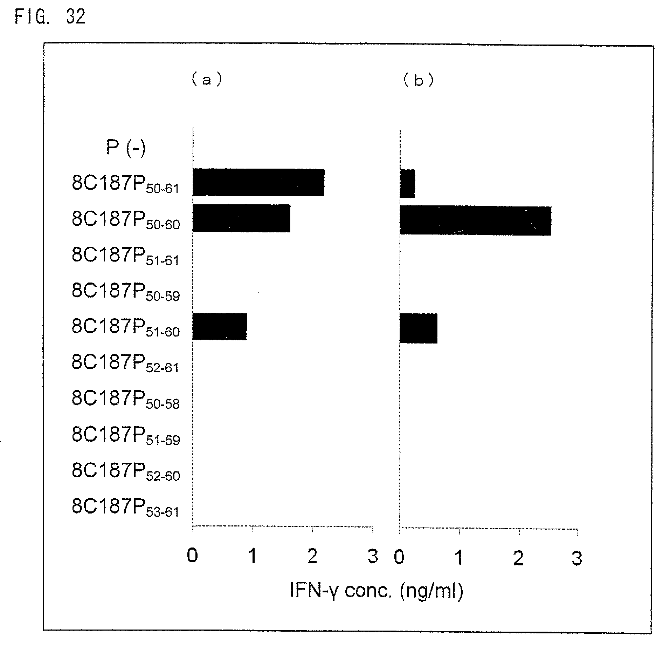

   Fig. 32 is a view illustrating a HLA-A*0206-restricted XAGE-1b zone recognized by CD8-positive T-cells; (a) is a view showing a result in the presence of serum, and (b) is a view showing a result in the absence of serum.
Fig. 33

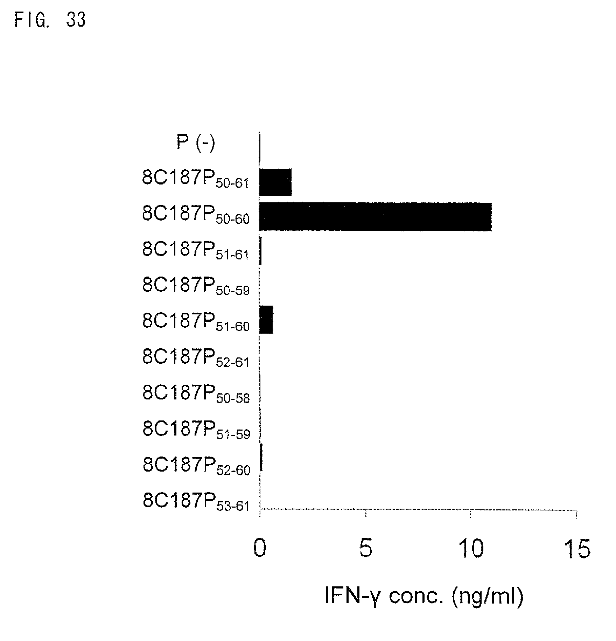

   Fig. 33 is a view illustrating a HLA-A*0206-restricted XAGE-1b zone recognized by CD8-positive T-cells.

### Description of Embodiments

Described below is an embodiment of the present invention. Note that the present invention is not limited to this embodiment.

### [1. Peptide according to the present invention]

A peptide according to the present invention is a peptide made up of an amino acid sequence of any one of the following (a) to (e), or is a peptide made up of an amino acid sequence of any one of the following (f) to (k):
(a) an amino acid sequence from position-1 to position-25 of SEQ ID. No. 1;
(b) an amino acid sequence from position-15 to position-53 of SEQ ID. No. 1;
(c) an amino acid sequence being a partial sequence of the amino acid sequence from position-15 to position-53 of SEQ ID. No. 1, the partial sequence including an amino acid sequence from position-15 to position-39, from position-29 to position-53, from position-21 to position-48, from position-21 to position-36, from position-25 to position-40, from position-29 to position-44, or from position-33 to position-48, each of SEQ ID. No. 1;
(d) an amino acid sequence from position-43 to position-81 of SEQ ID. No. 1;
(e) an amino acid sequence being a partial sequence of the amino acid sequence from position-43 to position-81 of SEQ ID. No. 1, the partial sequence including an amino acid sequence from position-43 to position-67, from position-57 to position-81, from position-57 to position-72, or from position-65 to position-81, each of SEQ ID. No. 1;
(f) an amino acid sequence from position-1 to position-39 of SEQ ID. No. 1;
(g) an amino acid sequence being a partial sequence of the amino acid sequence from position-1 to position-39 of SEQ ID. No. 1, the partial sequence being an amino acid sequence from position-1 to position-25, from position-15 to position-39, from position-13 to position-36, from position-13 to position-28, from position-17 to position-32, from position-21 to position-36, from position-9 to position-24, or from position-21 to position-29, each of SEQ ID. No. 1;
(h) an amino acid sequence from position-29 to position-53 of SEQ ID. No. 1;
(i) an amino acid sequence being a partial sequence of the amino acid sequence from position-29 to position-53 of SEQ ID. No. 1, the partial sequence including an amino acid sequence from position-29 to position-48, from position-29 to position-44, or from position-33 to position-48, each of SEQ ID. No. 1;
(j) an amino acid sequence from position-43 to position-81 of SEQ ID. No. 1; and
(k) an amino acid sequence being a partial sequence of the amino acid sequence from position-43 to position-81 of SEQ ID. No. 1, the partial sequence including an amino acid sequence from position-43 to position-67, from position-57 to position-81, from position-53 to position-68, from position-49 to position-64, from position-50 to position-60, or from position-51 to position-59, each of SEQ ID. No. 1.

A peptide made up of an amino acid sequence of any one of the amino acid sequences from position-21 to position-40, from position-21 to position-44, from position-25 to position-44, from position-25 to position-48, and from position-29 to position-48, each of SEQ ID. No. 1, are also encompassed in the peptides made up of the amino acid sequence of the foregoing (c). Moreover, a peptide made up of an amino acid sequence from position-13 to position-32 or from position-17 to position-36, each of SEQ ID. No. 1, is also encompassed in the peptides made up of the amino acid sequence of the foregoing (g).

The peptide according to the present invention can induce humoral immunity and cellular immunity against XAGE-1b in lung cancer. In the present specification, there may be cases where the peptides are distinguished, as a peptide made up of less than 15 amino acids being referred to as a "short chain peptide" and a peptide made up of 15 or more amino acids being referred to as a "long chain peptide".

Moreover, the peptide according to the present invention can be prepared by a conventionally known method of synthesizing a peptide. For example, an organic synthesis method such as solid phase peptide synthesis or the like may be used, or the peptide may be prepared with use of recombinant DNA techniques upon preparation of a nucleic acid which encodes the peptide.

The peptide according to the present invention, provided that the peptide can induce the humoral immunity and cellular immunity against XAGE-1b, encompasses peptides into which a variation such as deletion, substitution, addition, or insertion of one or several amino acids is introduced.

In the present specification, the term "peptide" is used exchangeable with polypeptide and protein, and is not limited in the number of amino acids which make up the peptide.

The International Journal of Oncology 30: 835-840, 2007 (Non-patent Literature 14) discloses, as a fragment of XAGE-1b, a polypeptide made up of an amino acid sequence from position-33 to position-49 or from position-25 to position-40 of the amino acid sequence of XAGE-1b (see Fig. 3(b) of Non-patent Literature 14). Moreover, Microbiol. Immunol., 51(8), 755-762, 2007 (Non-patent Literature 15) discloses, as a fragment of XAGE-1b, a polypeptide made up of an amino acid sequence from position-9 to position-24, from position-13 to position-28, from position-17 to position-32, from position-21 to position-36, from position-25 to position-40, from position-29 to position-44, from position-49 to position-64, from position-53 to position-68, from position-57 to position-72, or from position-65 to position-81, each of the amino acid sequence of XAGE-1b (see Fig. 2A of Non-patent Literature 15).

The present application does not intend to include these peptides in the scope of the "peptide" of the present invention. Namely, in an embodiment, a peptide according to the present invention may be a peptide with the proviso that the peptide is not any of the peptides disclosed in Non-patent Literature 14 and Non-patent Literature 15. In one aspect, a peptide according to the present embodiment is a peptide made up of an amino acid sequence of any one of the foregoing (a) to (e) (with the proviso that the peptide is not a peptide disclosed in Non-patent Literature 14 and Non-patent Literature 15), and in another aspect, a peptide according to the present embodiment is a peptide made up of an amino acid sequence of any one of the foregoing (f) to (k) (with the proviso that the peptide is not a peptide disclosed in Non-patent Literature 14 and Non-patent Literature 15).

The foregoing literatures neither disclose nor suggest functions of the peptide according to the present invention. Hence, the peptides disclosed in the foregoing literatures are not intended to be excluded from the scope of the present invention in terms of their utilization according to the present invention, later described. Namely, in one embodiment, a peptide according to the present invention is a peptide made up of an amino acid sequence of any one of the foregoing (a) to (e), or is a peptide made up of an amino acid sequence of any one of the foregoing (f) to (k).

In one aspect, a peptide according to the present embodiment is (i) a peptide made up of an amino acid sequence from position-15 to position-53 of SEQ ID. No. 1. In another aspect, a peptide according to the present embodiment is (ii) a peptide made up of an amino acid sequence from position-15 to position-39 (SEQ ID. No. 20), from position-29 to position-53 (SEQ ID. No. 21), from position-21 to position-48 (SEQ ID. No. 2), from position-21 to position-36 (SEQ ID. No. 3), from position-25 to position-40 (SEQ ID. No. 4), from position-29 to position-44 (SEQ ID. No. 5), or from position-33 to position-48 (SEQ ID. No. 6), each of SEQ ID. No. 1.

Moreover, since an effect of the peptide made up of the amino acid sequence from position-15 to position-53 of SEQ ID. No. 1 is maintained by a peptide made up of the amino acid sequence from position-15 to position-39, from position-29 to position-53, from position-21 to position-48, from position-21 to position-36, from position-25 to position-40, from position-29 to position-44, or from position-33 to position-48, each of SEQ ID. No. 1, a person skilled in the art who has read the present specification would easily understand that a peptide which is a fragment (partial fragment) of a peptide made up of the amino acid sequence from position-15 to position-53 of SEQ ID. No. 1 and which fragment includes any one of amino acid sequences from position-15 to position-39, from position-29 to position-53, from position-21 to position-48, from position-21 to position-36, from position-25 to position-40, from position-29 to position-44, and from position-33 to position-48, each of the amino acid sequence of SEQ ID. No. 1, is also encompassed within the scope of the present invention. Namely, the peptide according to the present embodiment may be (iii) a peptide made up of an amino acid sequence being a partial sequence of the amino acid sequence from position-15 to position-53 of SEQ ID. No. 1, the partial sequence including the amino acid sequence from position-15 to position-39, from position-29 to position-53, from position-21 to position-48, from position-21 to position-36, from position-25 to position-40, from position-29 to position-44, or from position-33 to position-48, each of SEQ ID. No. 1.

Furthermore, a person skilled in the art who has read the present specification would easily understand that with a peptide made up of the amino acid sequence from position-15 to position-53 of SEQ ID. No. 1, as long as the amino acid sequence from position-15 to position-39, from position-29 to position-53, from position-21 to position-48, from position-21 to position-36, from position-25 to position-40, from position-29 to position-44, or from position-33 to position-48, each of SEQ ID. No. 1 is maintained, the amino acid sequence in zones other than the foregoing zone may be more or less different. Namely, a peptide according to the present embodiment may be (iv) a peptide made up of an amino acid sequence or its partial sequence in which one or several amino acid is deleted from, substituted from or added to the amino acid sequence from position-15 to position-53 of SEQ ID. No. 1, the partial sequence including the amino acid sequence from position-15 to position-39, from position-29 to position-53, from position-21 to position-48, from position-21 to position-36, from position-25 to position-40, from position-29 to position-44, or from position-33 to position-48, each of SEQ ID. No. 1.

Each of the amino acid sequences from position-15 to position-53, from position-15 to position-39, from position-29 to position-53, from position-21 to position-48, from position-21 to position-36, from position-25 to position-40, from position-29 to position-44, and from position-33 to position-48, each of SEQ ID. No. 1, includes as a common amino acid sequence the amino acid sequence "from position-33 to position-36 of SEQ ID. No. 1". Accordingly, a person skilled in the art who has read the present specification would easily understand that the amino acid sequence from position-33 to position-36 of SEQ ID. No. 1 is a main zone for the peptide of the foregoing (i) through (iv) to function.

Similarly, in one aspect, the peptide according to the present embodiment is (i)' a peptide made up of an amino acid sequence from position-43 to position-81 of SEQ ID. No. 1. In another aspect, the peptide according to the present embodiment is (ii)' a peptide made up of an amino acid sequence from position-43 to position-67 (SEQ ID. No. 22), from position-57 to position-81 (SEQ ID. No. 23), from position-57 to position-72 (SEQ ID. No. 7), or from position-65 to position-81 (SEQ ID. No. 8), each of SEQ ID. No. 1. Furthermore, the peptide according to the present embodiment may be (iii)' a peptide made up of an amino acid sequence which is a partial sequence the amino acid sequence from position-43 to position-81 of SEQ ID. No. 1, the partial sequence including the amino acid sequence from position-43 to position-67, from position-57 to position-81, from position-57 to position -72, or from position-65 to position-81, each of SEQ ID. No. 1, or may be (iv)' a peptide made up of an amino acid sequence or its partial sequence in which one or several amino acid is deleted from, substituted from, or added to the amino acid sequence from position-43 to position-81 of SEQ ID. No. 1, the partial sequence being made up of the amino acid sequence from position-43 to position-67, from position-57 to position-81, from position-57 to position-72, or from position-65 to position-81, each of SEQ ID. No. 1.

Each of the amino acid sequences from position-43 to position-81, from position-43 to position-67, from position-57 to position-81, from position-57 to position-72, and from position-65 to position-81, each of SEQ ID. No. 1, has as a common amino acid sequence of "from position-65 to position-67 of SEQ ID. No. 1". Accordingly, a person skilled in the art who has read the present specification would easily understand that the amino acid sequence from position-65 to position-67 of SEQ ID. No. 1 is the main zone for the peptide of the foregoing (i)' through (iv)' to function.

Similarly, in one aspect, the peptide according to the present embodiment is (i)" a peptide of an amino acid sequence from position-1 to position-39 of SEQ ID. No. 1. In another aspect, the peptide according to the present embodiment is (ii)" a peptide made up of an amino acid sequence from position-1 to position-25 (SEQ ID. No. 19), from position-15 to position-39 (SEQ ID. No. 20), from position-13 to position-36 (SEQ ID. No. 9), from position-13 to position-28 (SEQ ID. No. 10), from position-17 to position-32 (SEQ ID. No. 11), from position-21 to position-36 (SEQ ID. No. 3), from position-9 to position-24 (SEQ ID. No. 14), or from position-21 to position-29 (SEQ ID. No. 15), each of SEQ ID. No. 1. Furthermore, the peptide according to the present embodiment may be (iii)" a peptide made up of an amino acid sequence which is a partial sequence of the amino acid sequence from position-1 to position-39 of SEQ ID. No. 1, the partial sequence including the amino acid sequence from position-1 to position-25, from position-15 to position-39, from position-13 to position-36, from position-13 to position-28, from position-17 to position-32, from position-21 to position-36, from position-9 to position-24, or from position-21 to position-29, each of SEQ ID. No. 1, or may be (iv)" a peptide made up of an amino acid sequence or its partial sequence in which one or several amino acid is deleted from, substituted from, or added to the amino acid sequence from position-1 to position-39 of SEQ ID. No. 1, the partial sequence including the amino acid sequence from position-1 to position-25, from position-15 to position-39, from position-13 to position-36, from position-13 to position-28, from position-17 to position-32, from position-21 to position-36, from position-9 to position-24, or from position-21 to position-29, each of SEQ ID. No. 1.

Each of the amino acid sequences from position-1 to position-39, from position-1 to position-25, from position-15 to position-39, from position-13 to position-36, from position-13 to position-28, from position-17 to position-32, from position-21 to position-36, from position-9 to position-24, and from position-21 to position-29, each of SEQ ID. No. 1, has as a common amino acid sequence of "an amino acid sequence from position-21 to position-24 of SEQ ID. No. 1". Hence, a person skilled in the art who has read the present specification would easily understand that the amino acid sequence from position-21 to position-24 of SEQ ID. No. 1 is the main zone for the peptide of (i)" through (iv)" to function.

Similarly, in one aspect, a peptide according to the present embodiment is (i)"' a peptide made up of an amino acid sequence from position-29 to position-53 of SEQ ID. No. 1. In another aspect, the peptide according to the present embodiment is (ii)"' a peptide made up of an amino acid sequence from position-29 to position-48 (SEQ ID. No. 12), from position-29 to position-44 (SEQ ID. No. 5), or from position-33 to position-48 (SEQ ID. No. 6), each of SEQ ID. No. 1. Furthermore, the peptide according to the present embodiment may be (iii)"' a peptide made up of an amino acid sequence which is a partial sequence of the amino acid sequence from position-29 to position-53 of SEQ ID. No. 1, the partial sequence including the amino acid sequence from position-29 to position-48, from position-29 to position-44, or from position-33 to position-48, each of SEQ ID. No. 1, or may be (iv)"' a peptide made up of an amino acid sequence or its partial sequence in which one or several amino acid is deleted from, substituted from or added to the amino acid sequence from position-29 to position-53 of SEQ ID. No. 1, the partial sequence being the amino acid sequence from position-29 to position-48, from position-29 to position-44, or from position-33 to position-48, each of SEQ ID. No. 1.

Each of the amino acid sequences from position-29 to position-53, from position-29 to position-48, from position-29 to position-44, and from position-33 to position-48, each of SEQ ID. No. 1, has as a common amino acid sequence of "from position-33 to position-44 of SEQ ID. No. 1". Accordingly, a person skilled in the art who has read the present specification would easily understand that the amino acid sequence from position-33 to position-44 of SEQ ID. No. 1 is the main zone for the peptide of (i)"' to (iv)"' to function.

Similarly, in one aspect, the peptide according to the present embodiment is (i)"" a peptide made up of an amino acid sequence from position-43 to position-81 of SEQ ID. No. 1. In another aspect, the peptide according to the present embodiment is (ii)"" a peptide made up of an amino acid sequence from position-43 to position-67 (SEQ ID. No. 22), from position-57 to position-81 (SEQ ID. No. 23), from position-53 to position-68 (SEQ ID. No. 13), from position-49 to position-64 (SEQ ID. No. 16), from position-50 to position-60 (SEQ ID. No. 17), or from position-51 to position-59 (SEQ ID. No. 18), each of SEQ ID. No. 1. Furthermore, the peptide according to the present embodiment may be (iii)"" a peptide made up of an amino acid sequence which is a partial sequence of the amino acid sequence from position-43 to position-81 of SEQ ID. No. 1, the partial sequence being the amino acid sequence from position-43 to position-67, from position-57 to position-81, from position-53 to position-68, from position-49 to position-64, from position-50 to position-60, or from position-51 to position-59, each of SEQ ID. No. 1, or may be (iv)"" a peptide made up of an amino acid sequence or its partial sequence in which one or several amino acid is deleted from, substituted from or added to the amino acid sequence from position-43 to position-81 of SEQ ID. No. 1, the partial sequence including the amino acid sequence from position-43 to position-67, from position-57 to position-81, from position-53 to position-68, from position-49 to position-64, from position-50 to position-60, or from position-51 to position-59, each of SEQ ID. No. 1.

Each of the amino acid sequences from position-43 to position-81, from position-43 to position-67, from position-57 to position-81, from position-53 to position-68, from position-49 to position-64, from position-50 to position-60, and from position-51 to position-59, each of SEQ ID. No. 1, includes a common amino acid sequence of "from position-57 to position-59 of SEQ ID. No. 1". Hence, a person skilled in the art who has read the present specification would easily understand that the amino acid sequence from position-57 to position-59 of SEQ ID. No. 1 is the main zone for the peptide of the foregoing (i)"" to (iv)"" to function.

Furthermore, as described later in Examples, the peptides respectively made up of the amino acid sequence from position-21 to position-36, from position-25 to position-40, from position-29 to position-44, and from position-33 to position-48, each of SEQ ID. No. 1, have a function to induce humoral immunity against lung cancer. Hence, a person skilled in the art who has read the present specification would easily understand that each of peptides made up of the amino acid sequence from position-15 to position-53, from position-15 to position-39, from position-29 to position-53, or from position-21 to position-48, each of SEQ ID. No. 1, which amino acid sequence includes the amino acid sequence from position-21 to position-36, from position-25 to position-40, from position-29 to position-44, or from position-33 to position-48, each of SEQ ID. No. 1, similarly would have this function, and that these peptides may also be encompassed within the scope of the present invention. Moreover, the peptides made up of the amino acid sequence of any one of amino acid sequences from position-21 to position-40, from position-21 to position-44, from position-25 to position-44, from position-25 to position-48, and from position-29 to position-48, each of SEQ ID. No. 1, also have a similar function as the peptide made up of the amino acid sequence from position-21 to position-48 of SEQ ID. No. 1, and these peptides may also be within the scope of the present invention.

Similarly, a peptide verified as having a function of inducing humoral immunity against lung cancer, which peptide is made up of the amino acid sequence from position-43 to position-81, from position-43 to position-67, or from position-57 to position-81, each of SEQ ID. No. 1, which amino acid sequence includes the amino acid sequence from position-57 to position-72 or from position-65 to position-81 of SEQ ID. No. 1, also has a similar function, and these peptides also may be encompassed within the scope of the present invention.

Moreover, as described later in Examples, peptides made up of an amino acid sequence from position-13 to position-28, from position-17 to position-32, from position-21 to position-36, from position-9 to position-24, or from position-21 to position-29, each of SEQ ID. No. 1, have a function for inducing cellular immunity against lung cancer. Hence, a person skilled in the art who has read the present specification would easily understand that a peptide made up of the amino acid sequence from position-1 to position-39, from position-1 to position-25, from position-15 to position-39, or from position-13 to position-36, each of SEQ ID. No. 1, which amino acid sequence includes the amino acid sequence from position-13 to position-28, from position-17 to position-32, from position-21 to position-36, from position-9 to position-24, or from position-21 to position-29, each of SEQ ID. No. 1, has a similar function, and that these peptides may also be encompassed within the scope of the present invention. Moreover, a peptide made up of an amino acid sequence from position-13 to position-32 or from position-17 to position-36, also has a similar function as the peptide made up of the amino acid sequence from position-13 to position-36 of SEQ ID. No. 1, and such a peptide may also be encompassed in the scope of the present invention.

Similarly, the peptide verified as having a function of inducing cellular immunity against lung cancer, which peptide is made up of an amino acid sequence from position-29 to position-53 or from position-29 to position-48, each of SEQ ID. No. 1, each of which includes the amino acid sequence from position-29 to position-44 or from position-33 to position-48 of SEQ ID. No. 1, also has a similar function, and these peptides may also be encompassed within the scope of the present invention.

Similarly, the peptide verified as having a function to induce cellular immunity against lung cancer, which peptide is made up of the amino acid sequence from position-43 to position-81, from position-43 to position-67, or from position-57 to position-81, each of SEQ ID. No. 1, each of which includes the amino acid sequence from position-53 to position-68, from position-49 to position-64, from position-50 to position-60, or from position-51 to position-59 of SEQ ID. No. 1, also has a similar function, and these peptides may also be encompassed within the scope of the present invention.

### [2. Composition according to the present invention for diagnosing lung cancer]

A composition according to the present invention for diagnosing lung cancer (hereinafter referred to as "composition 1 according to the present invention" or "composition 1") includes a peptide made up of the amino acid sequence of any one of the foregoing (a) through (e). The "peptide" is as described in "1. Peptide according to the present invention". The composition 1 according to the present invention may solely contain one peptide out of the foregoing peptides or may contain two or more peptides in combination.

Methods for diagnosing lung cancer with use of the composition 1 according to the present invention include, for example, radioimmunoassay (RIA), ELISA method (enzyme immunoassay), Western blotting, immunoprecipitation, immunohistochemistry, antibody array method, RT-PCR method, and real-time RT-PCR method. Accordingly, the composition 1 according to the present invention may include, other than the peptide, a buffer, salts, surfactants and the like that are regularly used in the foregoing diagnosis methods.

Moreover, non-small-cell lung cancer or lung adenocarcinoma may be diagnosed with use of the composition 1 according to the present invention.

### [3. Lung cancer diagnosis method according to the present invention]

A lung cancer diagnosis method according to the present invention is a method of diagnosing lung cancer, and includes measuring a level of peptide present in a sample derived from a subject (peptide measuring step), which peptide is made up of the amino acid sequence of any one of the foregoing (a) through (e), or includes measuring a level of an antibody specifically binding to the peptide in the sample derived from the subject (antibody measuring step), which peptide is made up of the amino acid sequence of any one of the foregoing (a) through (e). The "peptide" is as described in "1. Peptide according to the present invention".

The "subject" is not particularly limited and broadly includes animals in general. However, it is preferable that the subject is human. In a case where the subject is human, not only patients suffering from cancer and patients with the fear of having cancer, but also healthy persons may also serve as the subject. Sex, age and the like of the subject are not particularly limited. The "sample" may be, for example, blood (e.g. serum, plasma, blood cell), urine, feces, sputum, pleural effusion/ascitis, broncoalveolar lavage fluid, peritoneal washing, biopsy tissue, surgically-resected specimen etc. or the like. The "level of peptide present in a sample derived from a subject" denotes an amount of peptide present in a sample derived from the subject. The "level of an antibody" denotes an amount of the antibody.

The lung cancer diagnosis method according to the present invention, in the peptide measuring step or the antibody measuring step later described, determines by detecting whether or not a peptide made up of the amino acid sequence of any one of the foregoing (a) through (e) or an antibody specifically binding to these peptides is present in a sample derived from the subject. Moreover, cancer can be diagnosed by measuring the amount of peptide that is present in the sample derived from the subject, which peptide is made up of the amino acid sequence of any one of the foregoing (a) through (e), or be diagnosed by measuring the amount of the antibody present in the sample derived from the subject, which antibody is specifically binding to the peptides, and thereafter comparing this measured amount with a control (for example, the amount thereof present in a sample derived from a healthy body).

Moreover, with the lung cancer diagnosis method according to the present invention, it is possible to suitably diagnose non-small-cell lung cancer or lung adenocarcinoma, out of the lung cancers.

The following description deals with the "peptide measuring step" and "antibody measuring step".

### (3-1. Peptide measuring step)

The peptide measuring step is a step of measuring the amount of peptide in a sample derived from a subject, which peptide is made up of an amino acid sequence of any one of the foregoing (a) to (e). The amount of the peptide may be measured by use of methods known in the relative field. For instance, measurement may be carried out by radioimmunoassay (RIA), ELISA method (enzyme immunoassay), Western Blotting, immunoprecipitation, immunohistochemistry, antibody array method, RT-PCR method, real-time RT-PCR method or the like, with use of an antibody specifically recognizing the peptide made up of the amino acid sequence of any one of the foregoing (a) to (e). However, the present invention is not limited to this. The sample used for measuring the amount of the peptide is preferably serum, however as long as the amount of the peptide can be measured, the sample is not particularly limited.

The subject can be determined as having cancer in a case where the amount of the peptide present in the sample derived from the subject is higher than that in the control (e.g. the amount of the peptide present in the sample derived from a healthy body (healthy human)). The control may be obtained by measuring the sample derived from a normal healthy body simultaneously with the peptide measuring step, or alternatively, data accumulated as background data may be used as the control.

### (3-2. Antibody measuring step)

The antibody measuring step is a step of measuring a level of an antibody specifically binding to a peptide made up of the amino acid sequence of any one of the foregoing (a) to (e). The method of measuring a concentration of the antibody is not particularly limited as long as the method measures a level of antibiotic potency against a particular antigen or uses an antibody against a target antibody, and methods known in the relative field may be used. For instance, methods such as the ELISA method, radioimmunoassay, ELISPOT method, immunoprecipitation, affinity column method or the like may be used, however the present invention is not limited to these methods. The sample used for measuring the level of the antibody is preferably serum, however the sample is not particularly limited as long as the antibody level of the sample can be measured.

The antigen protein used for the measurement of the antibody potency may be obtained by refining an antigen protein from a living body sample, however it is preferable to obtain the antigen protein as a recombinant protein. The recombinant protein is obtained by introducing into a host an expression vector into which for example a XAGE-1b gene or a polynucleotide encoding an amino acid sequence of any one of the foregoing (a) to (e) is inserted, which host is then expressed and purified.

The subject can be determined as having cancer in a case where the amount of the antibody present in the sample derived from the subject is higher than that in the control (e.g. the amount of the antibody present in the sample derived from a healthy body (healthy human)). The control may be obtained by measuring the sample derived from a normal healthy body simultaneously with the antibody measuring step, or alternatively, data accumulated as background data may be used as the control. For example, in a case where ELISA is carried out with use of serum diluted 300 times, a healthy human (control) would not react. Hence, it is determined statistically as positive if absorbance (OD value) at a wavelength of 490 nm exceeds 1.0, which allows for determining that the subject has cancer. If the reaction condition such as the dilute strength of the serum is different, an OD value for determining the sample as positive changes. Accordingly, it is possible to appropriately change the settings in accordance with the reaction condition.

The lung cancer diagnosis method according to the present invention may be a method of obtaining data for diagnosing a possibility of the lung cancer. In this case, the present invention does not intend to include a determination step by a doctor.

### [4. Composition according to the present invention for inducing humoral immunity against lung cancer]

A composition according to the present invention for inducing humoral immunity against lung cancer (hereinafter referred to as "composition 2 according to the present invention" or "composition 2") contains a peptide made up of the amino acid sequence of any one of the foregoing (a) to (e). The "peptide" is as described in the foregoing "1. Peptide according to the present invention".

The composition 2 can induce humoral immunity against lung cancer as long as at least one type of peptide made up of an amino acid sequence of any one of the foregoing (a) to (e) are contained. However, by containing a plurality of peptides in combination, it is possible to induce humoral immunity against lung cancer more efficiently.

Moreover, the composition 2 according to the present invention may further contain another component other than a peptide, which component does not inhibit biological activity of the peptide (e.g. pharmaceutically acceptable carrier, etc.).

The "pharmaceutically acceptable carrier" in the present specification (hereinafter, also simply referred to as "carrier") is a substance used in aid of prescription when producing medicine or agrochemicals such as animal drugs, and which gives no harmful effect on active components of the pharmaceutical composition. Furthermore, the carrier intends to be a substance which has no toxicity against a body accepting the pharmaceutical composition according to the present invention, and which itself does not induce production of a harmful antibody.

Various organic or inorganic carrier substances may be used as the carrier, which substances are usable as pharmaceutical preparation material. These substances can be selected as appropriate according to administration forms and dosage forms of a pharmaceutical composition later described. For example, the various organic or inorganic carrier substances may be mixed therein as a diluent, lubricant, binding agent, disintegrator, or the like in a case of a solid preparation; a solvent, solubilizer, suspension, isotonizing agent, buffer, soothing agent, or the like in a case of a liquid preparation; anticeptics; antioxidizing agent; stabilizer; corrigents, or the like, however the present invention is not limited to these.

The composition 2 according to the present invention can induce humoral immunity against lung cancer by administering the composition 2 orally or parenterally, in the presence or absence of an adjuvant normally used in the medical and pharmaceutical field. In the present specification, the "parenteral" denotes a form of administration including injection or introduction conducted intraventricularly, intravenously, intramuscularly, intraperitoneally, infrasternally, subcutaneously, and intraarticularly.

Out of the lung cancers, the composition 2 according to the present invention can suitably induce humoral immunity against non-small-cell lung cancer or lung adenocarcinoma.

### [5.Composition according to the present invention for inducing cellular immunity against lung cancer]

A composition according to the present invention for inducing cellular immunity against lung cancer (hereinafter referred to as "composition 3 according to the present invention" or "composition 3 ") contains a peptide made up of the amino acid sequence of any one of the foregoing (f) to (k). The "peptide" is as described in the foregoing "1. Peptide according to the present invention".

The composition 3 which contains a peptide made up of (i) the amino acid sequence from position-1 to position-39 of SEQ ID. No. 1, (ii) the amino acid sequence being a partial sequence of the amino acid sequence from position-1 to position-39 of SEQ ID. No. 1, the partial sequence including the amino acid sequence from position-1 to position-25, from position-15 to position-39, from position-13 to position-36, from position-13 to position-28, from position-17 to position-32, or from position-21 to -36, each of SEQ ID. No. 1, (iii) the amino acid sequence from position-29 to position-53 of SEQ ID. No. 1, (iv) the amino acid sequence being a partial sequence of the amino acid sequence from position-29 to position-53 of SEQ ID. No. 1, the partial sequence including the amino acid sequence from position-29 to position-48, from position-29 to position-44, or from position-33 to position-48, each of SEQ ID. No. 1, (v) the amino acid sequence from position-43 to position-81 of SEQ ID. No. 1, or (vi) the amino acid sequence being a partial sequence of the amino acid sequence from position-43 to position-81 of SEQ ID. No. 1, the partial sequence including the amino acid sequence from position-43 to position-67, from position-57 to position-81 or from position-53 to position-68, each of SEQ ID. No. 1, can induce XAGE-1b-specific CD4-positive T-cells. Moreover, the composition 3 including a peptide made up of any one of amino acid sequences of (vii) the amino acid sequence from position-1 to position-39 of SEQ ID. No. 1, (viii) the amino acid sequence being a partial sequence of the amino acid sequence from position-1 to position-39 of SEQ ID. No. 1, the partial sequence including the amino acid sequence from position-1 to position-25, from position-15 to position-39, from position-21 to position-36, from position-9 to position-24, or from position-21 to position-29, each of SEQ ID. No. 1, (ix) the amino acid sequence from position-29 to position-53 of SEQ ID. No. 1, (x) the amino acid sequence being a partial sequence of the amino acid sequence from position-29 to position-53 of SEQ ID. No. 1, the partial sequence including the amino acid sequence from position-29 to position-44 of SEQ ID. No. 1, (xi) an amino acid sequence from position-43 to position-81 of SEQ ID. No. 1, and (xii) the amino acid sequence being a partial sequence of the amino acid sequence from position-43 to position-81 of SEQ ID. No. 1, the partial sequence including the amino acid sequence from position-43 to position-67, from position-57 to position-81, from position-49 to position-64, from position-50 to position-60, or from position-51 to position-59, each of SEQ ID. No. 1, can induce XAGE-1b-specific CD8-positive T-cells. Particularly, the composition 3 including a peptide made up of the amino acid sequence from position-50 to position-60 (SEQ ID. No. 17) of SEQ ID. No. 1 can induce HLA-A*0206-restricted XAGE-1b-specific CD8-positive T-cells. Moreover, the composition 3 including a peptide made up of the amino acid sequence from position-51 to position-59 (SEQ ID. No. 18) of SEQ ID. No. 1 can induce HLA-Cw*0102-restricted XAGE-1b-specific CD8-positive T-cells. Moreover, the composition 3 including a peptide made up of the amino acid sequence of a peptide made up of the amino acid sequence from position-21 to position-29 of SEQ ID. No. 1 (SEQ ID. No. 15) induces HLA-B*3501-restricted XAGE-1b-specific CD8-positive T-cells and HLA-B*4002-restricted XAGE-1b-specific CD8-positive T-cells.

The composition 3 can induce cellular immunity against lung cancer as long as at least one type of the peptides made up of the amino acid sequence of any one of the foregoing (f) to (k) is included. However, by including a plurality of peptides in combination, it is possible to induce the cellular immunity against lung cancer more efficiently.

Moreover, the composition 3 according to the present invention may further include another component other than the peptide (e.g. a pharmaceutically acceptable carrier, etc.), which component does not inhibit biological activity of the peptide. The "pharmaceutically acceptable carrier" is as described in "Composition according to the present invention for inducing humoral immunity against lung cancer", and thus is omitted in description here.

The composition 3 according to the present invention can induce cellular immunity against lung cancer by administering the composition 3 orally or paternally, in the presence or absence of an adjuvant normally used in the medical and pharmaceutical field.

Moreover, it is possible to induce the XAGE-1b-specific CD4-positive or CD8-positive T-cell in vitro by extracting a mononuclear cell fraction from peripheral blood of a lung cancer patient and co-culturing this with the composition including peptide made up of the amino acid sequence of any one of the foregoing (f) to (k). Prevention or treatment of lung cancer is possible also by injecting the induced XAGE-1b-specific T cell back into the blood of the lung cancer patient. In the case where the XAGE-1b-specific T cells are induced in vitro, the culturing conditions such as the density of the cells, concentration of the composition 3 and the like can be set as appropriate.

Out of the lung cancers, the composition 3 according to the present invention can preferably induce humoral immunity against non-small-cell lung cancer or lung adenocarcinoma.

As described in the Examples later described, although it was observed in the analysis of XAGE-1b that sites of XAGE-1b that are recognized by the specific antibody, specific CD4-positive T-cells, or the specific CD8-positive T-cells tend to be specific, the sites which were recognized by the specific antibody, specific CD4-positive T-cells, or specific CD8-positive T-cells were present along an entire length of XAGE-1b. Hence, in order to have the vaccine go into effect at low cost, regardless of the HLA with use of a long chain peptide which can induce antigen presentation to a degree similar to protein, it is preferable to administer the long chain peptide in combination so that the entire length of the XAGE-1b is covered.

The invention being thus described, it will be obvious that the same way may be varied in many ways. Such variations are not to be regarded as a departure from the spirit and scope of the invention, and all such modifications as would be obvious to one skilled in the art are intended to be included within the scope of the following claims.

### Examples

The following explains the present invention in more detail by describing Examples. It should be noted that the present invention is not limited to these Examples.

### [Example 1]

In Example 1, XAGE-1b antigen was analyzed in detail in terms of immunogenicity, and it was examined whether XAGE-1b antigen would be useful or not as a target cancer antigen in a new vaccine therapy for cancers.

### <Experiment material and experiment method>

### [a. Serums, pleural effusions, and peripheral blood mononuclear cells of patients]

Serums, pleural effusions, and peripheral blood mononuclear cells used in Examples of the present invention were offered as specimens from specimen donors with informed consents of all the specimen donors.

### [b. Method of separating cells]

Mononuclear cells were obtained from peripheral blood of a patient by density gradient centrifugation. Subsequently, using anti-CD4 antibody-binding beads, anti-CD8 antibody-binding beads, and anti-CD19 antibody-binding beads (Miltenyi Biotec), CD8-positive cells, CD4-positive cells, CD19-positive cells, and CD4-CD8-CD19-cells were sequentially separated by magnetic cell separation (MACS, Miltenyi Biotec).

### [c. XAGE-1b protein and peptide]

XAGE-1b protein (81 amino acids, SEQ ID. No. 1) used in the Example was one synthesized by GL Biochem (Shanghai, China). The synthesized XAGE-1b protein was purified in HPLC column, and was confirmed that its purity was 90 % or more.

Overlapping peptide (which may be hereinafter abbreviated as "OLP") of 16-mer or 17-mer used in the Example, which covered the entire length of XAGE-1b protein (1-16, 5-20, 9-24, 13-28, 17-32, 21-36, 25-40, 29-44, 33-48, 37-52, 41-56, 45-60, 49-64, 53-68, 57-72, 61-76, and 65-81) was one synthesized using a multiple peptide synthesizer (AMS422; ABIDED, Langenfeld Germany) by a Fmoc solid phase method in the joint laboratory of Okayama University.

### [d. ELISA]

Full-length XAGE- 16 protein (carbonic acid buffer, pH 9.6) with a concentration of 1 µg/ml was immobilized overnight on a 96 well plate (NUNC) at 4 °C, and then the plate was washed with a washing solution (PBS/0.1 % TWEEN). To the washed plate, 5 % FCS/PBS was added and the plate was subjected to blocking at 37 °C for 1 hour. After the blocking, serum diluted 100 times, 300 times, 900 times, or 2700 times was added and the resultant was caused to react at 37 °C for 2 hours. Subsequently, the resultant was washed with the washing solution, and then peroxidase-binding goat anti-human IgG antibody (diluted 5000 times) (Jackson ImmunoResearch Laboratories, Inc.) was added to the resultant and this mixture was caused to react at 37 °C for 1 hour. After the reaction, the resultant was washed with the washing solution and a substrate solution to which hydrogen peroxide was added (solution obtained by dissolving orthophenylendiamine in 0.05M citric acid buffer (pH 5.0)) was added to stain the resultant. After the staining, 6N sulfuric acid was added to stop the reaction, and absorbance (wavelength of 490 nm) thereof was measured using a microplate reader (Bio-Rad Laboratories, Inc.).

### [e. IFN-γ catch assay]

The following explains IFN-γ catch assay with reference to Fig. 6. Fig. 6 is a view showing a procedure of IFN-γ catch assay.

### (1) Induction of XAGE-1b-specific reaction in CD4-positive or CD8-positive T cells

CD4-positive or CD8-positive T cells and an equal number of X-ray-irradiated (70 Gy) CD4-CD8-T cells which served as antigen-presenting cells ("APC" in the drawings) were cultured for 10-14 days in a CO₂ incubator using a 24 well plate or a 96 well plate each in the presence of 1 µM of XAGE-1b overlapping peptide (OLP). A culture medium for culturing the T cells was 5 % pool serum/AIM-V (IL-2 25IU/ml, IL-7 5ng/ml) unless otherwise stated, and 5 µg/ml of IL-15 was added if necessary.

According to necessity, a second stimulation was made similarly in such a manner that half of the culture media were replaced with new ones and XAGE-1b overlapping peptide was added so that the amount of the XAGE-1b overlapping peptide would be 1 µM.

The amount of antigen-specific IFN-γ produced by T cells 10-14 days after the first or second stimulation was measured by ELISA below.

### (2) IFN-γ ELISA

Mouse anti-human IFN-γ monoclonal antibody (1-D1K, Becton, Dickinson and Company, diluted 500 times) was immobilized overnight on a plate and was blocked. To this plate, 100 µl of stimulated culture supernatant of effecter cells (CD4-positive or CD8-positive T cells) was added, and the resultant was caused to react at 37°C for 1 hour. Thereafter, the plate was washed with PBST (0.1 % Tween 20-PBS), rabbit anti-human IFN-γ antibody (own-made, diluted 600 times) was added and the resultant was caused to react at 37°C for 1 hour.

After washing the plate with a washing solution, HRP binding goat anti-rabbit IgG antibody (MBL, diluted 2000 times) was added and the resultant was caused to react at 37°C for 1 hour. After the reaction, the plate was washed with the washing solution and then a substrate solution (o-phenylenediamine, OPDA) (Wako Pure Chemical Industries, Ltd.) was added to stain the resultant. After the staining, 6N sulfuric acid was added to stop the reaction, and absorbance (wavelength of 490 nm) thereof was measured using a microplate reader (Bio-Rad Laboratories, Inc.). A well where a large amount of IFN-γ was produced as compared to a well which was not stimulated with overlapping peptide was regarded as a positive well. Next day, with respect to the positive well, the presence of cells which produced IFN-γ in an antigen-specific manner were confirmed.

### (3) Detection of IFN-γ production cells

Cells having been stimulation-cultured with addition of overlapping peptides were caused to react with an equal number of self EBV-B cells (Epstein-Barr virus-infected B cells) (stimulated or not stimulated in advance with XAGE-1b overlapping peptide) at 37°C for 4 or 8 hours in a CO₂ incubator, and the cultured cells were marked with 2 µl of human IFN-γ catch antibody (Miltenyi Biotec).

Thereafter, the cultured cells were suspended in 1-10 ml AIM-V culture media, and were caused to react in the CO₂ incubator at 37 °C for 45 minutes while suspending the cultured cells by a rotator (MACSmix, Miltenyi Biotec). After washing the cells, the cells were stained with 2 µl of PE-marked human IFN-γ antibody (Miltenyi Biotec), 2 µl of 7AAD (Becton, Dickinson and Company), or 1 µl of FITC-marked anti-human CD4 antibody or FITC-marked anti-human CD8 antibody (Miltenyi Biotec).

After the staining, the cells were washed with FACS buffer (1 % FCS/PBS, 0.02 % sodium azide), and were subjected to flow cytometry using FACS Calibur (Becton, Dickinson and Company), to detect IFN-γ production cells. Frequency of the IFN-γ production cells was analyzed using data analysis software (FlowJo, Tree Star).

Fig. 1 is a view showing expression of mRNA of XAGE-1 in non-small-cell lung cancers. (a) of Fig. 1 shows analysis on expression of mRNAs of XAGE-la, -1b, -1c, and -1d which were four splicing variants of XAGE-1, in 10 cases of specimens (tissues) of lung adenocarcinoma and in 12 cases of lung cancer cell lines. As shown in (a) of Fig. 1, in the lung cancer cells, high expressions were observed in all XAGE-1 (1c and 1d were dominant), whereas in the lung cancer tissues, expressions of only XAGE-1b and XAGE-1d were observed. Non-patent Literatures 7 and 8 demonstrate that out of XAGE-1b and XAGE-1d, XAGE-1b is significant in lung cancers. Accordingly, it is desirable that XAGE-1b is a target of a candidate vaccine. Although the cause of a difference in the expression of XAGE-1 between the lung cancer cell lines and the tissues is not known, the difference may be related to the function of XAGE-1.
(b) of Fig. 1 demonstrates the presence of XAGE-1b protein in lung cancer cell lines. Specifically, (b) of Fig. 1 demonstrates the presence of protein in mRNA-positive lung cancer cell lines by Western blotting. It should be noted that a difference in the amount of expression between lung cancer cell lines and lung cancer tissues has not been examined so far, and the inventors of the present invention were the first persons to disclose the difference. Since the amount of the expression of XAGE-1b protein in the lung cancer cell lines was extremely smaller than that in the lung cancer tissues, identification of XAGE-1b protein in the lung cancer cell lines was only possible by immunoprecipitation.

### [Test Example 1]

Whether or not humoral immune responses to XAGE-1b were present were examined for 200 cases of subjects suffering from non-small-cell lung cancer (including 69 cases of subjects suffering from lung adenocarcinoma in the late stage) who had consulted Kawasaki Medical School Hospital from 2005 to 2009, and for 50 cases of normal healthy subjects serving as controls.

Specifically, whether or not XAGE-1b-specific IgG antibody was present in serums sampled from lung cancer patients was examined by ELISA.

The results of ELISA are shown in Figs. 2 and 3. Fig. 2 is a view showing antibody responses to XAGE-1b protein in the patients suffering from non-small-cell lung cancer. (a) of Fig. 2 shows absorbance (OD values) measured by ELISA of serums (diluted 100 times, 300 times, 900 times, and 2,700 times) of the patients suffering from non-small-cell lung cancer, and (b) of Fig. 2 shows absorbance (OD values) measured by ELISA of serums (diluted 100 times, 300 times, 900 times, and 2,700 times) of the normal healthy subjects. Fig. 3 is a view showing zones of antibody responses to XAGE-1b proteins in the patients suffering from non-small-cell lung cancer.

As shown in Figs. 2 and 3, the lung cancer patients were classified into four zones of a very-strong zone, a strong zone, a weak zone, and a border zone in the order of serum antibody titer, compared with the control group (normal healthy subject). Among them, the lung cancer patients of the very-strong zone, the strong zone, and the weak zone were regarded as serum antibody titer-positive patients.

Out of all the non-small-cell lung cancer patients, cases in which the patient was XAGE-1b serum antibody titer-positive counted 20/200 (10.0 %). As shown in Table 1, when focused just on the lung adenocarcinoma patients in the late stage (stage 3B/4), cases in which the patient was XAGE-1b serum antibody titer-positive counted 13/69 (18.8%).

**Table 1**

| | Non-small-cell lung cancer (n = 200) | Stage 3B/4 lung adenocarcinoma (n = 69) |
|---|---|---|
| n (%) | 20 (10.0 %) | 13 (18.8 %) |

On the other hand, the same tests were carried out to the 50 cases of normal healthy subjects. However, as shown in (b) of Fig. 2, no antibody reaction to XAGE-1b was observed. These results show that among the lung cancer patients, the non-small-cell lung cancer patients, particularly lung adenocarcinoma patients, had highly frequent induction of humoral immunity to XAGE-1b.

Non-patent Literature 13 reports that 32.5 % of lung adenocarcinoma was tissue immunostaining-positive with respect to XAGE-1b. From this fact, it is expected that when tissue immunostaining is positive with respect to XAGE-1b in lung adenocarcinoma in the late stage, humoral immunity to XAGE-1b will be induced with a high frequency of approximately 60 %.

### [Test Example 2]

Using serums of patients who were serum antibody titer-positive, epitope was examined of XAGE-1b which was recognized by anti-XAGE-1b antibody contained in the serums. Fig. 4 is a view showing an amino acid sequence of XAGE-1b overlapping peptides used in epitope analysis.

Seventeen kinds of XAGE-1b overlapping peptides shown in Fig. 4 were immobilized on plates each with a concentration of 1 µg/ml, and serums of serum antibody titer-positive patients were added to the plates. Thereafter, the presence of an antigen-antibody reaction was examined by ELISA. The serums used here were diluted 300 times.

The results of examining 20 cases of serum antibody titer-positive patients by ELISA are shown in Figs. 5 and 6. Fig. 5 is a view showing antibody recognition with respect to XAGE-1b overlapping peptide. The broken line in the graph of Fig. 5 indicates an antibody titer (O.D. (490 nm)) of 0.1. Fig. 6 is a view showing zones of XAGE-1b which were recognized by anti-XAGE-1b antibodies. In Fig. 6, the number of patients whose antibody titers were 0.1 or more in Fig. 5 was plotted with respect to each peptide.

As shown in Fig. 5, zones recognized by anti-XAGE-1b antibodies vary among individuals. However, as shown in Fig. 6, it is found that main sites recognized by anti-XAGE-1b antibodies were the following three zones: a zone from position-21 to position-48 (SEQ ID. No. 2), a zone from position-57 to position-72 (SEQ ID. No. 7), and a zone from position-65 to position-81 (SEQ ID. No. 8), each in the full-length amino acid sequence of XAGE-1b.

### [Test Example 3]

A reaction to XAGE-1b in peripheral blood CD4-positive T cells was examined. Peripheral blood CD4-positive T cells (1 × 10⁶ cells) were separated from each of 16 serum antibody titer-positive patients. The peripheral blood CD4-positive T cells thus separated were stimulation-cultured for 10-14 days with an equal number of CD4-CD8-cells irradiated with radioactive rays, in the presence of overlapping peptides which were pooled to cover the entire length of XAGE-1b (stimulation-culturing was carried out two times per 10-14 days according to necessity). Subsequently, 1 × 10⁴ CD4-positive T cells and an equal number of PFA-treated self-EBV-B cells that were pulsed or not pulsed with XAGE-1b overlapping peptides were caused to react at 37 °C for 4 hours. IFN-γ catch assay was carried out to the resultant.

As a result, as shown in (a) and (b) of Fig. 8, in 14 patients out of 16 serum antibody titer-positive patients (87.5 %), cells which produced IFN-γ in an XAGE-1b-specific manner were detected out of the CD4-positive T cells stimulated with XAGE-1b overlapping peptides. The percentage of the detected specific T cells was 87.5 % (14/16) in CD4.

Fig. 30 is a view showing responses of CD4-positive T cells to XAGE-1b peptides in two typical cases where reaction was observed. (a) of Fig. 30 shows the result of flow cytometry, and (b) of Fig. 30 shows the result of measuring serum antibody titer of patients by ELISA. As shown in (a) of Fig. 30, in case KLU34, 1.11 % (net) of IFN-γ production cells were detected. In case KLUN38, 0.94 % (net) of IFN-γ production cells were detected. The term "net" indicates a value obtained by subtracting the percentage of IFN-γ production cells in peptide (+) from the percentage of IFN-γ production cells in peptide (-).

### [Test Example 4]

The 14 patients from which XAGE-1b-specific CD4-positive T cells were detected in Test Example 3 were examined as to the zones of XAGE-1b which were recognized by the specific CD4-positive T cells. 1 × 10⁴ CD4-positive T cells which had been obtained in the foregoing tests were caused to react with, as antigen-presenting cells, an equal number of PFA-treated self-EBV-B cells pulsed or not pulsed with 5 µg/ml of XAGE-1b overlapping peptides, at 37 °C for 4 hours. Thereafter, IFN-γ catch assay or ELISA was carried out thereto.

The result is shown in Fig. 31. Fig. 31 is a view showing zones of XAGE-1b which were recognized by the stimulation-cultured XAGE-1b-specific CD4-positive T cells of the serum antibody titer-positive patient (KLU38). As shown in Fig. 31, when the CD4-positive T cells were stimulated with peptide 4 (amino acid zone from position-13 to position-28, SEQ ID. No. 10) or peptide 6 (amino acid zone from position-21 to position-36, SEQ ID. No. 3), cells which produced IFN-γ in an XAGE-1b specific manner were detected highly frequently. This indicates that the CD4-positive T cells in case KLU38 recognized the amino acid zone from position-13 to position-36 and the amino acid zone from position-21 to position-36 of XAGE-1b.

The remaining 13 cases of the serum antibody titer-positive patients were examined similarly. The results of the examinations are shown in Figs. 9 and 10. Fig. 9 is a view showing zones of XAGE-1b which were recognized by the specific CD4-positive T cells (n = 14). In Fig. 9, peptides to which the specific CD4-positive T cells reacted particularly strongly are marked with stars. Fig. 10 is a view showing main epitope zones of XAGE-1b overlapping peptides which were recognized by the XAGE-1b specific CD4-positive T cells (n = 14). In Fig. 10, the number of stars in Fig. 9 is plotted with respect to each peptide. From the results of examining the 14 cases of the XAGE-1b antibody-positive shown in Figs. 9 and 10, it was found that the amino acid sequences from position-13 to position-36 (SEQ ID. No. 9), from position-29 to position-48 (SEQ ID. No. 12), and from position-53 to position-68 (SEQ ID. No. 13), each of the XAGE-1b amino acid sequence, were recognized highly frequently by the XAGE-1b-specific CD4-positive T cells. Further, it was found that main sites recognized by the XAGE-1b-specific CD4-positive T cells were the amino acid zone from position-13 to position-28 (SEQ ID. No. 10) and the amino acid zone from position-33 to position-48 (SEQ ID. No. 6).

### [Test Example 5]

In order to examine zones of the CD8-positive T cells which recognize XAGE-1b, peripheral blood CD8-positive T cells were subj ected to IFN-γ catch assay and ELISA. Specifically, 1 × 10⁴ peripheral blood CD8-positive T cells obtained from serum antibody-positive patients were cultured together with an equal number of CD4-CD8-cells in the presence of 1 µg/ml of XAGE-1b overlapping peptides. On 10-14 days after the start of the culturing, the amount of XAGE-1b-specific IFN-γ produced by the CD8-positive T cells was measured by ELISA. As a result of IFN-γ catch assay, reactions of XAGE-1b-specific CD8-positive T cells were observed in 6 cases out of 9 cases of the serum antibody titer-positive patients (66.7 %). (c) and (d) of Fig. 8 are views showing induction of the XAGE-1b-specific CD8-positive T cells.

### [Test Example 6]

In order to examine zones of XAGE-1b which are recognized by the CD8-positive T cells, the 6 cases exhibiting reactions were subjected to ELISA using self-EBV-B cells as the antigen-presenting cells, which self-EBV-B cells were pulsed with 1 µg/ml of individual XAGE-1b overlapping peptides. The result is shown in Fig. 11. Fig. 11 is a view showing the zones of XAGE-1b which were recognized by the specific CD8-positive T cells (n = 6). In Fig. 11, peptides to which the specific CD8-positive T cells respond particularly strongly are marked with stars. Fig. 12 is a view showing main epitope zones recognized by the XAGE-1b-specific CD8-positive T cells (n = 6). In Fig. 12, the number of stars in Fig. 11 is plotted with respect to each peptide. From the results of examining the 6 cases of XAGE-1b antibody-positive shown in Figs. 11 and 12, it was found that the amino acid sequences from position-9 to position-24 (SEQ ID. No. 14), from position-21 to position-36 (SEQ ID. No. 3), from position-29 to position-44 (SEQ ID. No. 5), and from position 49 to position-64 (SEQ ID. No. 16), each of the XAGE-1b amino acid sequence, were recognized highly frequently by the XAGE-1b-specific CD8-positive T cells.

Fig. 13 is a view showing main epitope zones recognized by the XAGE-1b-specific antibodies, the XAGE-1b-specific CD4-positive T cells, or the XAGE-1b-specific CD8-positive T cells in the serum antibody titer-positive patients. As shown in Fig. 13, in the analysis of XAGE-1b, although it is observed that sites of XAGE-1b recognized by the specific antibody, specific CD4-positive T-cells, or the specific CD8-positive T-cells tend to be specific, the sites which were recognized by the specific antibody, specific CD4-positive T-cells, or specific CD8-positive T-cells were present along the entire length of XAGE-1b. Hence, it was considered that by combining a plurality of peptides so that the entire length of the XAGE-1b is covered and by administering this combined peptide, it was possible to realize an XAGE-1b vaccine regardless of a kind of HLA of a subject.

Fig. 14 shows a relation of main epitope zones with HLA, which main epitope zones are recognized by XAGE-1b-specific CD4-positive T-cells and CD8-positive T-cells.

Fig. 14 shows HLA of 15 cases in which XAGE-1b specific T cells could be detected and zones of overlapping peptides which were recognized by the specific T cells. Black indicates zones recognized by the CD4-positive T cells and gray indicates zones recognized by the CD8-positive T cells. The cancer vaccine that the inventors of the present invention intend is one which can be used regardless of HLA of the subject. As described above, the reaction specific to XAGE-1b is present along the entire length of XAGE-1b, and among the entire length, there are main zones recognized by the antibody, the CD4-positive T cells and the CD8-positive T cells. Further, as described later, the inventors of the present invention succeeded in identifying epitope peptides restricted by several kinds of HLA. Since there are many kinds of human HLA as shown in Fig. 14, identification of epitopes for individual kinds of HLA would be very difficult. Accordingly, a table showing correspondences between HLAs of individual subjects and main recognition zones as shown in Fig. 14 would be useful for supposing a new epitope peptide. There is a possibility in the future that a peptide zone will be found, which peptide zone allows for inducing a specific reaction based on a certain combination of HLAs, very efficiently.
(a) of Fig. 15 shows the result of IFN-γ ELISA, which shows the result of peptide recognition by 8C187-1. As shown in (a) of Fig. 15, when CD8-positive T cells (8C187-1) were stimulated with a peptide made up of an amino acid sequence from position-45 to position-60 of the XAGE-1b amino acid sequence (peptide 45-60) or a peptide made up of an amino acid sequence from position-49 to position-64 of the XAGE-1b amino acid sequence (peptide 49-64), the CD8-positive T cells produced IFN-γ. This expressly indicates that clone T cells (8C187-1) established from case KLU187 recognized the amino acid sequence from position-45 to position-60 and the amino acid sequence from position-49 to position-64 of the XAGE-1b amino acid sequence. Further, (b) of Fig. 15 shows the results of IFN-γ ELISA with addition of an anti-CD4 antibody, an anti-CD8 antibody, an anti-class I antibody, or an anti-class II antibody. As shown in (b) of Fig. 15, it was found that the T cells recognize peptides in a CD8-restricted manner and a class I-restricted manner.

In order to examine which peptides presented by what kind of HLA is recognized by the CD8-positive T cells, IFN-γ ELISA was carried out with use of various kinds of EBV B cells as antigen-presenting cells, with respect to the peptide made up of the amino acid sequence from position-49 to position-64 of the XAGE-1b amino acid sequence (peptide 49-64). The results are shown in (c) of Fig. 15. (c) of Fig. 15 shows that HLA-A*0206-restricted CD8-positive T cell clones recognized this peptide. As shown in (c) of Fig. 15, when CD8-positive T cell clones (8C187-1) obtained from antigen-specific CD8-positive T cells of KLU187 were stimulated with EBV-B cells expressing HLA-A*0206 (self EBV-B cells and OS-P07 EBV-B cells of KLU187) as antigen-presenting cells, cells were detected which produced IFN-γ in an XAGE-1b-specific manner. From this result, it was found that HLA-A*0206-restricted CD8-positive T cell clones (8C187-1) recognized the peptide 49-64.

Further, with respect to the peptides 45-60 and 49-64, in order to determine the minimum epitope zone recognized by HLA-A*0206-restricted XAGE-1b-specific CD8-positive T cells, various peptides shown in Fig. 16 (48-61, 49-61, 50-61, 51-61, 52-61, 53-61, 49-64, 48-61, 48-60, 48-59, 48-58, 48-57, and 48-56) were synthesized and IFN-γ ELISA was carried out.

The result is shown in Fig. 16. Fig. 16 is a view showing zones of XAGE-1b which were recognized by HLA-A*0206-restricted CD8-positive T cells. As shown in (a) and (b) of Fig. 16, from the result of IFN-γ ELISA, it was expected that the minimum epitope recognized by CD8-positive T cells is to be made up of 11 amino acids.

Since serum contains proteolytic enzyme etc., it is supposed that various components and enzymes in the serum modify peptides. Therefore, the same analysis was carried out in the absence of serum. Conditions for IFN-γ ELISA were as follows:
KLU187 CD8 (8C187-1) 1 × 10⁴ cells
Peptide: XAGE-1b peptide 1 µM for each.

The result of IFN-γ ELISA is shown in Fig. 32. As shown in (b) of Fig. 32, P50-60 exhibited the strongest response also in the absence of serum (i.e. in the absence of proteolytic enzyme etc.) (AIM-V). This confirmed that the epitope zone is from position-50 to position-60 of the XAGE-1b amino acid sequence. Further, according to the result shown in (a) of Fig. 32, the epitope seems to be P50-61 in the presence of serum (5% PS/AIM-V), but P50-61 exhibited a reduced response in the absence of serum. On the other hand, P50-60 exhibited a significant response also in the absence of serum. From these results, it is inferred that the epitope is from position-50 to position-60 (SEQ ID. No. 17) of the XAGE-1b amino acid sequence.

Further, in order to determine an epitope zone presented by HLA-A*0206, IFN-γ ELISA was carried out using non-self antigen-presenting cells (Mi-EBV-B) which share just HLA-A*0206 and HLA-Cw*0102. It was supposed that T cells would exhibit a reduced response in the absence of serum. Accordingly, in order to maintain activity of the T cells, IL-2 was added to the AIM-V culture medium. Conditions for IFN-γ ELISA were as follows:
KLU187 CD8 (8C187-1) 1 × 10⁴ cells
Peptide: XAGE-1b peptide 1 µM for each.

The result of IFN-γ ELISA is shown in Fig. 33. As shown in Fig. 33, when the CD8-positive T cell clones were stimulated with P50-60, the CD8-positive T cell clones produced IFN-γ. This indicates that the CD8-positive T cell clones recognized P50-60 presented on HLA-A*0206. As shown in (c) of Fig. 15, the CD8-positive T cell clones did not recognize peptides presented by (Okazaki EBV-B) HLA-Cw*0102. This confirms that out of the antigen-presenting cells (Mi-EBV-B) which share HLA-A*0206 and HLA-Cw*0102, only the peptide presented by HLA-A*0206 was recognized by the CD8-positive T cell clones.

As shown in Fig. 18 later described, peptides normally recognized by CD8-positive T cells are often made up of 9 amino acids. In view of this, peptides made up of 9-12 amino acids including the amino acids from position-50 to position-60 of the XAGE-1b amino acid sequence (P50-61, P50-60, P51-61, and P51-60) were prepared, and it was examined whether the 11 amino acid sequences from position-50 to position-60 of the XAGE-1b amino acid sequence were really the minimum epitope. The result of IFN-γ ELISA is shown in (c) of Fig. 16.

As shown in (c) of Fig. 16, it was found that when CD8-positive T cells were stimulated in the absence of serum with a peptide including the amino acid sequence from position-50 to position-60 (SEQ ID. No. 17) of the XAGE-1b amino acid sequence, the CD8-positive T cells produced an increased amount of IFN-γ in a peptide-concentration-dependent manner. Further, stimulation of the CD8-positive T cells with a peptide including an amino acid sequence from position-50 to position-60 of the XAGE-1b amino acid sequence exhibited a remarkable effect on production of IFN-γ, compared with stimulation of the CD8-positive T cells with a peptide including an amino acid sequence from position-50 to position-60 of the XAGE-1b amino acid sequence. Since the response of the CD8-positive T cells was maintained even when the peptide had low concentration, it was found that the peptide presented by HLA-A*0206 is made up of 11 amino acid sequences from position-50 to position-60 of the XAGE-1b amino acid sequence.

Further, after confirmation of CD8-restrictedness and class I-restrictedness of another T cell clones (8C187-2) obtained from the antigen-specific T cells of KLU187, the minimum epitope was determined in the same manner.

Fig. 17 is a view showing that the peptide 45-60 and the peptide 49-64 are peptides recognized by HLA-Cw*0102-restricted CD8-positive T cell clones (8C187-2); (a) of Fig. 17 indicates the result of IFN-γ ELISA, which shows the result of peptide recognition by 8C187-2, (b) of Fig. 17 shows the result of IFN-γ ELISA with addition of an anti-CD4 antibody, an anti-CD8 antibody, an anti-class I antibody, or an anti-class II antibody, and (c) of Fig. 17 shows that HLA-Cw*0102-restricted CD8-positive T cell clones recognize this peptide.

As shown in Fig. 17, when CD8-positive T cell clones (8C187-2) obtained from KLU187 were stimulated with EBV-B cells expressing HLA-Cw*0102 as antigen-presenting cells, production of XAGE-1b-specific IFN-γ was detected. From this result, it was found that HLA-Cw*0102-restricted CD8-positive T cell clones (8C187-2) recognize the peptide 49-64.

Further, in order to determine the minimum epitope zone recognized by HLA-Cw*0102-restricted XAGE-1b-specific CD8-positive T cell clones (8C187-2), several kinds of peptides shown in Fig. 18 (peptides 50-61, 51-61, 52-61, 50-60, 50-59 and 50-58) were synthesized and IFN-γ ELISA was carried out thereto.

The result is shown in Fig. 18. Fig. 18 is a view showing zones of XAGE-1b which were recognized by HLA-Cw*0102-restricted CD8-positive T cells. (c) of Fig. 18 is a view showing that the amount of IFN-γ produced by the CD8-positive T cells increased in a peptide-concentration-dependent manner.

It was expected that the peptide zone of XAGE-1b recognized by HLA-Cw*0102-restricted T cells be from position-51 to position-59 (SEQ ID. No. 18) of the XAGE-1b amino acid sequence. However, since the response of the CD8-positive T cells to P51-61 was very strong, the inventors of the present invention examined how CD8-positive T cell clones responded depending on concentrations of each of four kinds of peptides shown in (c) of Fig. 18 (peptides 51-61, 51-60, 51-59, and 50-59) with use of antigen-presenting cells (Mi-EBV-B) sharing just HLA-A*0206 and HLA-Cw*0102. The result of IFN-γ ELISA is shown in (c) of Fig. 18.

As shown in (c) of Fig. 18, it was found that when the CD8-positive T cell clones were stimulated with P51-60 or P51-61 which includes P51-59 being the minimum unit of HLA-Cw*0102-restricted peptide and to which C-terminus amino acid was added, the amount of IFN-γ produced by the CD8-positive T cell clones increased in a peptide-concentration-dependent manner similarly with the case of stimulation with P51-59. This demonstrates that the minimum epitope presented by HLA-Cw*0102 is from position-51 to position-59 (SEQ ID. No. 18) of the XAGE-1b amino acid sequence. Although there were a plurality of peptide zones presented by HLA-Cw*0102, it was supposed that a part serving as a core of the plurality of peptide zones was the amino acid sequence from position-51 to position-59 (SEQ ID. No. 18), excluding the amino acid at position-50, of the XAGE-1b amino acid sequence.

Fig. 19 is a view showing that peptide 21-36 is a peptide recognized by T cell clones (8C187-4). (a) of Fig. 19 shows the result of IFN-γ ELISA, which shows the result of peptide recognition by 8C187-4. (b) of Fig. 19 shows the result of IFN-γ ELISA with addition of an anti-CD4 antibody, an anti-CD8 antibody, an anti-class I antibody, or an anti-class II antibody. (c) of Fig. 19 shows that HLA-B*3501-restricted CD8-positive T cell clones recognize this peptide.

In (b) of Fig. 19, after confirmation of CD8-restrictedness and class I-restrictedness of another T cell clones (8C187-4) obtained from the antigen-specific T cells of KLU187, the minimum epitope was determined in the same manner. As shown in (c) of Fig. 19, when CD8-positive T cell clones (8C187-4) obtained from KLU187 were stimulated with EBV-B cells expressing HLA-B*3501 as antigen-presenting cells, production of XAGE-1b-specific IFN-γ were detected. From this result, it was found that HLA-B*3501-restricted CD8-positive T cell clones (8C187-4) recognized peptide 21-36.

In order to determine the minimum epitope zone recognized by HLA-B*3501-restricted XAGE-1b-specific CD8-positive T cells, a plurality of peptides shown in Fig. 20 (peptides 17-32, 21-36, 17-31, 18-31, 19-31, 20-31, 21-31, 21-30, 21-29, and 22-32) were synthesized and IFN-γ ELISA was carried out thereto.

The result is shown in Fig. 20. The minimum epitope zone recognized by HLA-B*3501-restricted XAGE-1b-specific CD8-positive T cell clones (8C187-4) was identified as being from position-21 to position-29 of the XAGE-1b amino acid sequence.

Fig. 21 is a view showing that peptide 21-36 is a peptide recognized by T cell clones (8C237-22). (a) of Fig. 21 shows the result of IFN-γ ELISA, which shows the result of peptide recognition by 8C237-22, (b) of Fig. 21 shows the results of IFN-γ ELISA with addition of an anti-CD4 antibody, an anti-CD8 antibody, an anti-class I antibody, or an anti-class II antibody, and (c) of Fig. 21 shows that HLA-B*4002-restricted CD8-positive T cell clones recognize this peptide.

In (b) of Fig. 21, after confirmation of CD8-restrictedness and class I-restrictedness of another T cell clones (8C237-22) obtained from the antigen-specific T cells of KLU237, the minimum epitope was determined in the same manner. As shown in (c) of Fig. 21, when CD8-positive T cell clones (8C237-22) obtained from KLU187 were stimulated with EBV-B cells expressing HLA-B*4002 as antigen-presenting cells, production of XAGE-1b-specific IFN-γ was detected. From this result, it was found that HLA-B*4002-restricted CD8-positive T cell clones (8C237-22) recognize the peptide 21-36.

In order to determine the minimum epitope zone recognized by HLA-B*4002-restricted XAGE-1b-specific CD8-positive T cell clones, a plurality of peptides shown in Fig. 22 (peptides 17-32, 21-36, 17-31, 18-31, 19-31, 20-31, 21-31, 21-30, 21-29, and 22-32) were synthesized and IFN-γ ELISA was carried out thereto.

The result is shown in Fig. 22. The minimum epitope zone recognized by HLA-B*4002-restricted XAGE-1b-specific CD8-positive T cell clones (8C237-22) was identified as being from position-21 to position-29 (SEQ ID. No. 15) of the XAGE-1b amino acid sequence.

Fig. 23 shows that stimulation of established XAGE-1b-specific CD4-positive T cells (4C187-1) with self-dendritic cells pulsed with XAGE-1b protein, lysate of 293T cells to which XAGE-1b plasmid DNA had been transfected, or lysate of self-lung cancer tissue, resulted in showing a reaction of production of antigen-specific IFN-γ.

Fig. 24 shows that stimulation of HLA-B*35-restricted CD8-positive T cell clones (8C187-4) with self-dendritic cells pulsed with XAGE-1b protein or five kinds of 25-mer XAGE-1b overlapping peptides (as for specific amino acid sequences, see Fig. 26) resulted in showing reaction of production of antigen-specific IFN-γ. Further, demonstration of the fact that the established T cells could react to 25-mer long-chain peptide demonstrated usefulness of the later-explained long-chain peptide. Further, (b) of Fig. 24 shows that 8C187-4 specifically recognized B*3501-positive tumor cells to which XAGE-1b plasmid DNA had been transfected.

As shown in Fig. 24, it was found that T cells obtained from an anti-XAGE-1b antibody-positive patient recognize natural epitope peptide and have cytotoxic activity specific to antigen-positive tumor cells. This suggests that XAGE-1b has strong immunogenicity and so a vaccine therapy targeting XAGE-1b is useful for non-small-cell lung cancer patients.

Induction of XAGE-1b-specific CD8-positive clone T cells had not been successfully done by anyone in the world until the inventors of the present invention succeeded in the induction. Further, peptide zones recognized by XAGE-1b-specific CD8-positive T cells had not been found until the present invention revealed the zones.

Further examined was a time which an antigen was reacted with the cells, to induce an immune reaction specific to XAGE-1b. Fig. 25 is a view showing induction of the immune reaction specific to XAGE-1b, in accordance with the time which the antigen was reacted with the cells. (a) of Fig. 25 shows the results in the presence of IL-2 and IL-7, and (b) and (c) of Fig. 25 show the results in the presence of IL-7 and IL-15. Further, (b) of Fig. 25 is a view showing that CD8-positive T cells producing IFN-γ in an XAGE-1b-specific manner was induced after 8-hour co-culturing with EBV-B cells that are stimulated with XAGE-1b overlapping peptides. (c) of Fig. 25 shows that also in a case of antigen-specific T cell clones, 8-hour co-culturing resulted in the reaction. Conditions for IFN-γ ELISA in (a)-(c) of Fig. 25 were as follows:
(a) of Fig. 25
   Effector: KLU187 CD8 1 × 10⁴ cells
   Target: KLU187 EBV-B (self) 2 × 10³ cells
   Peptide: XAGE-1b overlapping peptide 1 µg/ml
   After three-times of stimulation-culturing (IVS × 3)
(b) of Fig. 25
   Effector: KLU187 CD8 1 × 10⁴ cells
   Target: KLU187EBV-B (self) 1 × 10⁴ cells
   Peptide: XAGE-1b overlapping peptide 1 µg/ml
(c) of Fig. 25
   Effector: KLU187 CD8 clone 8C187-1 1 × 10⁴ cells
   Target: KLU187 EBV-B (self) 2 × 10³ cells
   Peptide: XAGE-1b overlapping peptide 1 µg/ml
   After three-times of stimulation-culturing (IVS × 3)

As shown in Fig. 25, it was found that even when IL-15 is added to further activate CD8-positive T cells or to strengthen antigen presenting ability, it is necessary to set the time for the response between antigen and CD8-positive T cells to 8 hours or so in order to induce a cell immune specific to XAGE-1b in a cancer patient to which a vaccine has not been administered.

Also with respect to NY-ESO-1 which is a publicly known CT antigen, CD8-positive T cells having been stimulation-cultured were caused to react with an equal number of self-EBV-B cells pulsed or not pulsed with NY-ESO-1 overlapping peptides in a CO₂ incubator at 37 °C for 4 hours to carry out IFN-γ catch assay, and it was confirmed that NY ESO-1-specific CD4-positive T cells or NY ESO-1-specific CD8-positive T cells can be detected (not shown in drawings).

Such a difference in the reaction time between the antigen and CD8-positive T cells seems to be derived from various causes such as the structure of a peptide and antigenicity of XAGE-1b. One possible cause is a difference in reaction mechanism of T cells to various cancer antigens.

### <Conclusion>

### [Results]

### [1. Humoral immune response]

For 200 examples of non-small-cell lung cancer patients, an immune reaction to XAGE-1b which is one of cancer testis antigens was analyzed in detail. The results were as follows:
(1) Antibody-positive cases counted 20/200 (10.0%) out of the whole cases of non-small-cell lung cancer patients.
(2) Antibody-positive cases were observed with a high frequency of 13/69 (18.8 %) out of cases of stage 3B/4 lung adenocarcinoma in the late stage.
(3) As a result of analysis on an immune reaction to NY ESO-1 for comparison, it was found that 6.7 % out of the whole cases of lung cancers were antibody-positive, and 9.7 % out of cases of stage 3/4 non-small-cell lung cancers were antibody-positive.
(4) In view of the above, it was found that a positive ratio of an antibody reaction to XAGE-1b was comparable with a positive ratio of an antibody response to NY ESO-1 which is already used as a vaccine target antigen in clinical tests, and XAGE-1b could be a candidate of a vaccine target antigen for lung cancer patients.
(5) It was found that the zones of XAGE-1b which are recognized by antibodies are the following three zones: a zone from position-21 to position-48 (SEQ ID. No. 2), a zone from position-57 to position-72 (SEQ ID. No. 7), and a zone from position-65 to position-81 (SEQ ID. No. 8), each of the XAGE-1b amino acid sequence.
(6) Further improvement in antibody detecting sensitivity would enable this finding to be applicable to diagnosis of lung cancers.

### [2. Cellular immune reaction]

Further analysis on immune reactions of XAGE-1b-specific CD4-positive T cells and XAGE-1b-specific CD8-positive T cells showed the following results:
(1) In analysis of 16 serum antibody titer-positive patients, XAGE-1b-specific CD4-positive T cells were successfully detected in 14 patients (87.5 %).
(2) It was found that zones recognized by CD4-positive T cells, where a specific reaction had been observed, were from position-13 to position-36 (SEQ ID. No. 9), from position-29 to position-48 (SEQ ID. No. 12), and from position-53 to position-68 (SEQ ID. No. 13), and main zones out of these zones were from position-13 to position-28 (SEQ ID. No. 10) and from position-33 to position-48 (SEQ ID. No. 6).
(3) Further, XAGE-1b-specific CD8-positive T cells were successfully induced and a method for detecting XAGE-1b-specific CD8-positive T cells was established.
(4) In analysis of 6 serum antibody titer-positive patients, XAGE-1b-specific CD8-positive T cells were successfully detected in 4 patients (66.7 %).
(5) It was found that the HLA-A*0206-restricted epitope zone recognized by specific CD8-positive T cells is from position-50 to position-60 (SEQ ID No. 17) of the XAGE-1b amino acid sequence, and that the HLA-Cw*0102-restricted epitope zone recognized by specific CD8-positive T cells is from position-51 to position-59 (SEQ ID No. 18) of the XAGE-1b amino acid sequence. Further, it was found that the HLA-B*3501-restricted epitope zone recognized by specific CD8-positive T cells and the HLA-B*4002-restricted epitope zone recognized by specific CD8-positive T cells is from position-21 to position-29 (SEQ ID No. 15) of the XAGE-1b amino acid sequence.
(6) Identification of epitope zones recognized by specific CD8-positive T cells enables peptides including these zones to be candidates for cancer peptide vaccines, thereby inducing cytotoxic T cells more efficiently.

### [Considerations]

The following points were considered in the present invention:
(1) Humoral and cellular responses of a lung cancer patient specific to XAGE-1b were confirmed.
(2) XAGE-1b antibody-positive cases were 20 (10.0 %) out of the whole 200 cases of non-small-cell cancers, and humoral immunes were induced in 13 cases (18.8 %) out of 69 cases of lung adenocarcinoma in the late stage (stage 3B/4).
(3) Zones recognized by anti-XAGE-1b antibody were the following three zones: a zone from position-21 to position-48 (SEQ ID. No. 2), a zone from position-57 to position-72 (SEQ ID. No. 7), and a zone from position-65 to position-81 (SEQ ID. No. 8), each of the XAGE-1b amino acid sequence.
(4) XAGE-1b-specific CD4-positive T cells were induced in 14 cases out of 16 cases (87.5 %). Zones of XAGE-1b which were recognized by CD4-positive T cells were from position-13 to position-36 (SEQ ID. No. 9), from position-29 to position-48 (SEQ ID. No. 12), and from position-53 to position-68 (SEQ ID. No. 13), each of the XAGE-1b amino acid sequence. Further, main zones were from position-13 to position-28 (SEQ ID. No. 10) and from position-33 to position-48 (SEQ ID. No. 6);
(5) XAGE-1b-specific CD8-positive T cells were induced in 4 cases out of 6 cases (66.7 %). Zones of XAGE-1b which are recognized by CD8-positive T cells were from position-9 to position-24 (SEQ ID. No. 14), from position-21 to position-36 (SEQ ID. No. 3), from position-29 to position-44 (SEQ ID. No. 5), and from position-49 to position-64 (SEQ ID. No. 16), each of the XAGE-1b amino acid sequence.
(6) It is difficult to detect XAGE-1b-specific CD8 T cells by a conventional method since the response between XAGE-1b and CD8 positive T cells takes a longer time than the response between NY ESO-1 etc. and CD8 positive T cells.
(7) A peptide zone recognized by HLA-A*0206-restricted CD8-positive T cells specific to XAGE-1b is from position-50 to position-60 (SEQ ID. No. 17) of the XAGE-1b amino acid sequence.
(8) A peptide zone recognized by HLA-Cw*0102-restricted CD8-positive T cells specific to XAGE-1b was from position-51 to position-59 (SEQ ID. No. 18) of the XAGE-1b amino acid sequence.
(9) A peptide zone recognized by HLA-B*3501-restricted CD8-positive T cells specific to XAGE-1b and recognized by HLA-B*4002-restricted CD8-positive T cells specific to XAGE-1b was from position-21 to position-29 (SEQ ID. No. 15) of the XAGE-1b amino acid sequence.
(10) Identification of zones recognized by XAGE-1b-specific antibodies allows for applying the present invention to diagnosis for lung cancers.
(11) Identification of zones recognized by XAGE-1b-specific CD4-positive T cells and XAGE-1b-specific CD8-positive T cells enables peptides including the zones to serve as candidates for peptides (antigens) used in a cancer vaccine therapy.
(12) Identification of zones recognized by XAGE-1b-specific CD8-positive T cells reveals that the zones are peptides with strong immunogenicity capable of inducing cytotoxic T cells. The peptides are applicable to cancer treatments including a cancer vaccine therapy.

### [Example 2]

In Example 2, long-chain peptides of XAGE-1b were prepared. Fig. 26 is a view showing amino acid sequences of long-chain peptides of XAGE-1b. As shown in Fig. 26, the long-chain peptides of XAGE-1b prepared in Example 2 are five kinds of peptides below each made up of 25 amino acids and designed to cover the entire length of XAGE-1b made up of 81 amino acids.
Peptide 1-25 (made up of an amino acid sequence of SEQ ID. No. 19)
Peptide 15-39 (made up of an amino acid sequence of SEQ ID. No. 20)
Peptide 29-53 (made up of an amino acid sequence of SEQ ID. No. 21)
Peptide 43-67 (made up of an amino acid sequence of SEQ ID. No. 22)
Peptide 57-81 (made up of an amino acid sequence of SEQ ID. No. 23)

Here described is the result of analysis on a peptide vaccine targeting NY-ESO-1 which is a cancer testis antigen likewise XAGE-1b.

As for NY-ESO-1 vaccine, a protein (NY-ESO-1 full length peptide) vaccine has been already put into use. However, it is assumed that NY ESO-1 protein vaccine have the following defects:
(i) the NY ESO-1 protein vaccine is very expensive;
(ii) since protein is a very large substance, antigen-presenting cells are difficult to be pulsed with the protein without an appropriate adjuvant (immunostimulant); and
(iii) since protein is an extraneous substance, the protein is less likely to be sufficiently presented as an antigen to CD8-positive T cells (less likely to be presented as an antigen via MHC class I).
Accordingly, at present, NY-ESO-1 protein zones with high immunogenicity (zones frequently recognized by specific antibody, specific CD4-positive T cells, or specific CD8-positive T cells) are estimated and a long-chain peptide vaccine containing such zones (NY-SO-1 f peptide vaccine) is put into use.

However, in a case where f peptide (long-chain peptide made up of 20 amino acids) is used as a vaccine, the following two points had been unknown:
(A) whether f peptide can be presented as an antigen; and
(B) whether f peptide vaccine exhibits the same effect as a protein vaccine.

Initially, the inventors of the present invention analyzed the point (A). Specifically, U937, which is a human monocytic leukemia strain, was subjected to priming using 20 ng/ml of PMA, and then cultured with NY-ESO-1 f peptide conjugated with FAM^{TM}. Localization of NY-ESO-1 f peptide in U937 was confirmed by observing fluorescence of FAM^{TM} which is a fluorescent pigment.

The result is shown in Fig. 27. Fig. 27 is a view illustrating that NY ESO-1 f peptide was taken into an antigen-presenting cell (U937). WGA (Wheat Germ Agglutini) in Fig. 27 indicates that cell membrane was stained.

As shown in Fig. 27, it was confirmed that NY ESO-1 f peptide was taken into the cells 3 hours after the start of culturing. From this result, it was found that NY ESO-1 f peptide (20 amino acids) shorter than NY ESO-1 protein (NY ESO-1 full length peptide) can be taken into the antigen-presenting cells.

Further, it was confirmed that NY ESO-1 f peptide can be presented as an antigen by MHC class II (Fig. 28). Specifically, NY ESO-1-specific CD4-positive T cell clones (E-8A1) were subjected to IFN-γ ELISA by use of self-EBV-B cells as antigen-presenting cells, in the presence of peptide NY ESO-1 f peptide at a temperature condition of 4 °C or 37 °C.

Fig. 28 is a view illustrating that NY ESO-1 f peptide is presented as an antigen by MHC class II. "E/T ratio" of the horizontal axis of Fig. 28 indicates a ratio of co-culturing between Effector (T cell) and Target (EBV-B cell). As shown in Fig. 28, it was found that presentation of NY-ESO-1 f peptide by MHC class II is influenced by temperature. This result indicates that although NY ESO-1 f peptide is presented as an antigen by MHC class II, intake of NY ESO-1 f peptide is blocked by a low temperature, thereby resulting in decrease in a reaction to CD4-positive T cells.

Further, it was confirmed that NY-ESO-1 f peptide is presented as an antigen also by MHC class I, i.e., cross-presented (Fig. 29). Specifically, in a non-serum AIM-V culture medium containing 1 µM of NY-ESO-1₉₂₋₁₀₀ short-chain peptide, 1 µM of NY-ESO-1₉₁₋₁₁₀ f peptide or 10 µg/ml of recombinant protein, dendritic cells (5 × 10⁵ cells/ml) were cultured and pulsed with antigen, in the presence/absence of 10 µM of cytochalasin B. The cells were washed and then CD8-positive T cell clones (TK-f01 2H10) (5 × 10³ cells) were cultured at 37 °C for 24 hours with the dendritic cells (5 × 10³ cells) pulsed with the antigens. The amount of production of IFN-γ by antigen stimulation was measured by ELISA.

Fig. 29 is a view illustrating that NY ESO-1 f peptide is presented as an antigen by MHC class I. As shown in Fig. 29, it was found that presentation of NY ESO-1 f peptide by MHC class I is influenced by cytochalasin B. This result indicates that although NY ESO-1 f peptide is presented as an antigen by MHC class I, intake of NY ESO-1 f peptide is blocked by cytochalasin B, thereby resulting in decrease in a reaction to CD8-positive T cells.

When an extraneous antigen (protein and peptide) is taken into an antigen-presenting cell, the extraneous antigen is presented as an antigen basically via an MHC class II route (route which contributes to induction of CD4-positive T cells). In contrast thereto, an MHC class I route (route which contributes to induction of CD8-positive T cells) is a route for presenting an endogeneous antigen as an antigen. However, there are cases where an extraneous antigen is presented as an antigen via the MHC class I route. This is known as the cross-presentation.

It was confirmed that NY ESO-1 f peptide (long-chain peptide) (i) is taken into an antigen-presenting cell, (ii) can activate CD4-positive T cells via the MHC class II route which is an original route for presenting an extraneous antigen as antigen, and (iii) can activate CD8-positive T cells by cross-presenting NY ESO-1 f peptide via the MHC class I route. Further, the result of Fig. 29 suggests that unless a suitable adjuvant is used, protein cannot sufficiently induce CD8-positive T cells by cross-presentation. These results demonstrate that a long-chain peptide has, as a vaccine, functions similar to those of protein.

Short-chain peptide vaccine (in a case of MHC class I route, the minimum epitope is made up of about 9-11 amino acids, e.g. vaccine prepared under the supervision of Professor Yusuke Nakamura, Tokyo University) is restricted by a kind of HLA of a subject. In contrast thereto, a protein vaccine is not restricted by the kind of HLA of a subject since the protein vaccine covers the entire length of an antigen.

Specifically, a short-chain peptide vaccine uses a peptide restricted by a specific HLA, and therefore cannot basically recognize other HLA-restricted T cells. For example, in a case of XAGE-1b, HLA-Cw*0102-restricted epitope peptide is a peptide including an amino acid sequence corresponding to position-51 to position-59 of the XAGE-1b amino acid sequence (peptide made up of 9 amino acids represented by SEQ ID. No. 18). The vaccine of this short-chain peptide can induce HLA-Cw*0102-restricted immune, but cannot induce HLA-A*0206-restricted immune. This is because HLA-A*0206-restricted minimum epitope is a peptide containing an amino acid sequence corresponding to position-50 to position-60 of the XAGE-1b amino acid sequence (peptide made up of 11 amino acids represented by SEQ ID. No. 17), and so the peptide made up of 9 amino acids represented by SEQ ID. No. 18 is too short for the HLA-A*0206-restricted minimum epitope. Consequently, the short-chain peptide vaccine made up of 9 amino acids represented by SEQ ID. No. 18 can only be used for a subject having an HLA named Cw0102.

In contrast thereto, for example, the short-chain peptide vaccine made up of 11 amino acids represented by SEQ ID. No. 17 can induce HLA-A*0206-restricted immune. Further, this short-chain peptide vaccine can be taken into an antigen-presenting cell and treated appropriately to induce HLA-Cw*0102-restricted immune. Namely, by identifying a zone (if possible, epitope peptide) recognized by a specific CD4-positive T cell or a specific CD8-positive T cell and examining the frequency of recognition, it is possible to prepare a long-chain peptide which can be an inexpensive and effective vaccine regardless of the kind of HLA of a subject, similarly with a protein vaccine.

As shown in Fig. 13, in the analysis of XAGE-1b, although it is observed that sites of XAGE-1b that are recognized by the specific antibody, specific CD4-positive T-cell, or the specific CD8-positive T-cell tend to be specific, the sites which are recognized by the specific antibody, specific CD4-positive T-cell, or specific CD8-positive T-cell are present along the entire length of XAGE-1b. Hence, it is considered that by combining a plurality of long-chain peptides so that the entire length of the XAGE-1b is covered and by administering this combined peptide, it is possible to realize an XAGE-1b vaccine regardless of the kind of HLA of a subject.

### Industrial Applicability

The present invention is usable for examination or diagnosis of cancer, prevention of cancer, treatment of cancer and the like, and is contributive not just to the development of medical science and medical service, but also to utilization in the clinical diagnostic agent industry, reagent industry and like industries.

## Claims

1. A peptide comprising an amino acid sequence of any one of the following (a) through (e) (with the proviso that the peptide is not a peptide disclosed in the International Journal of Oncology 30: 835-840, 2007 and Microbiol. Immunol., 51(8), 755-762, 2007):
(a) an amino acid sequence from position-1 to position-25 of SEQ ID. No. 1;
(b) an amino acid sequence from position-15 to position-53 of SEQ ID. No. 1;
(c) an amino acid sequence being a partial sequence of the amino acid sequence from position-15 to position-53 of SEQ ID. No. 1, the partial sequence including an amino acid sequence from position-15 to position-39, from position-29 to position-53, from position-21 to position-48, from position-21 to position-36, from position-25 to position-40, from position-29 to position-44, or from position-33 to position-48, each of SEQ ID. No. 1;
(d) an amino acid sequence from position-43 to position-81 of SEQ ID. No. 1; and
(e) an amino acid sequence being a partial sequence of the amino acid sequence from position-43 to position-81 of SEQ ID. No. 1, the partial sequence including an amino acid sequence from position-43 to position-67, from position-57 to position-81, from position-57 to position-72, or from position-65 to position-81, each of SEQ ID. No. 1.

2. A composition for diagnosing lung cancer comprising:
a peptide comprising an amino acid sequence of any one of the following (a) through (e):
(a) an amino acid sequence from position-1 to position-25 of SEQ ID. No. 1;
(b) an amino acid sequence from position-15 to position-53 of SEQ ID. No. 1;
(c) an amino acid sequence being a partial sequence of the amino acid sequence from position-15 to position-53 of SEQ ID. No. 1, the partial sequence including an amino acid sequence from position-15 to position-39, from position-29 to position-53, from position-21 to position-48, from position-21 to position-36, from position-25 to position-40, from position-29 to position-44, or from position-33 to position-48, each of SEQ ID. No. 1;
(d) an amino acid sequence from position-43 to position-81 of SEQ ID. No. 1; and
(e) an amino acid sequence being a partial sequence of the amino acid sequence from position-43 to position-81 of SEQ ID. No. 1, the partial sequence including an amino acid sequence from position-43 to position-67, from position-57 to position-81, from position-57 to position-72, or from position-65 to position-81, each of SEQ ID. No. 1.

3. The composition according to claim 2, wherein the lung cancer is non-small-cell lung cancer or lung adenocarcinoma.

4. A method of diagnosing lung cancer comprising measuring a level of an antibody binding specifically to a peptide in a sample derived from a subject, the peptide comprising an amino acid sequence of any one of the following (a) through (e):
(a) an amino acid sequence from position-1 to position-25 of SEQ ID. No. 1;
(b) an amino acid sequence from position-15 to position-53 of SEQ ID. No. 1;
(c) an amino acid sequence being a partial sequence of the amino acid sequence from position-15 to position-53 of SEQ ID. No. 1, the partial sequence including an amino acid sequence from position-15 to position-39, from position-29 to position-53, from position-21 to position-48, from position-21 to position-36, from position-25 to position-40, from position-29 to position-44, or from position-33 to position-48, each of SEQ ID. No. 1;
(d) an amino acid sequence from position-43 to position-81 of SEQ ID. No. 1; and
(e) an amino acid sequence being a partial sequence of the amino acid sequence from position-43 to position-81 of SEQ ID. No. 1, the partial sequence including an amino acid sequence from position-43 to position-67, from position-57 to position-81, from position-57 to position-72, or from position-65 to position-81, each of SEQ ID. No. 1.

5. A method of diagnosing lung cancer comprising measuring a level of peptide present in a sample derived from a subject, the peptide comprising an amino acid sequence of any one of the following (a) through (e):
(a) an amino acid sequence from position-1 to position-25 of SEQ ID. No. 1;
(b) an amino acid sequence from position-15 to position-53 of SEQ ID. No. 1;
(c) an amino acid sequence being a partial sequence of the amino acid sequence from position-15 to position-53 of SEQ ID. No. 1, the partial sequence including an amino acid sequence from position-15 to position-39, from position-29 to position-53, from position-21 to position-48, from position-21 to position-36, from position-25 to position-40, from position-29 to position-44, or from position-33 to position-48, each of SEQ ID. No. 1;
(d) an amino acid sequence from position-43 to position-81 of SEQ ID. No. 1; and
(e) an amino acid sequence being a partial sequence of the amino acid sequence from position-43 to position-81 of SEQ ID. No. 1, the partial sequence including an amino acid sequence from position-43 to position-67, from position-57 to position-81, from position-57 to position-72, or from position-65 to position-81, each of SEQ ID. No. 1.

6. A composition for inducing humoral immunity against lung cancer, the composition comprising a peptide comprising an amino acid sequence of any one of the following (a) through (e):
(a) an amino acid sequence from position-1 to position-25 of SEQ ID. No. 1;
(b) an amino acid sequence from position-15 to position-53 of SEQ ID. No. 1;
(c) an amino acid sequence being a partial sequence of the amino acid sequence from position-15 to position-53 of SEQ ID. No. 1, the partial sequence including an amino acid sequence from position-15 to position-39, from position-29 to position-53, from position-21 to position-48, from position-21 to position-36, from position-25 to position-40, from position-29 to position-44, or from position-33 to position-48, each of SEQ ID. No. 1;
(d) an amino acid sequence from position-43 to position-81 of SEQ ID. No. 1; and
(e) an amino acid sequence being a partial sequence of the amino acid sequence from position-43 to position-81 of SEQ ID. No. 1, the partial sequence including an amino acid sequence from position-43 to position-67, from position-57 to position-81, from position-57 to position-72, or from position-65 to position-81, each of SEQ ID. No. 1.

7. The composition according to claim 6, wherein the lung cancer is non-small-cell lung cancer or lung adenocarcinoma.

8. A peptide comprising an amino acid sequence of any one of the following (f) through (k) (with the proviso that the peptide is not a peptide disclosed in the International Journal of Oncology 30: 835-840, 2007 and Microbiol. Immunol., 51(8), 755-762, 2007):
(f) an amino acid sequence from position-1 to position-39 of SEQ ID. No. 1;
(g) an amino acid sequence being a partial sequence of the amino acid sequence from position-1 to position-39 of SEQ ID. No. 1, the partial sequence including an amino acid sequence from position-1 to position-25, from position-15 to position-39, from position-13 to position-36, from position-13 to position-28, from position-17 to position-32, from position-21 to position-36, from position-9 to position-24, or from position-21 to position-29, each of SEQ ID. No. 1;
(h) an amino acid sequence from position-29 to position-53 of SEQ ID. No. 1;
(i) an amino acid sequence being a partial sequence of the amino acid sequence from position-29 to position-53 of SEQ ID. No. 1, the partial sequence including an amino acid sequence from position-29 to position-48, from position-29 to position-44, or from position-33 to position-48, each of SEQ ID. No. 1;
(j) an amino acid sequence from position-43 to position-81 of SEQ ID. No. 1;
(k) an amino acid sequence being a partial sequence of the amino acid sequence from position-43 to position-81 of SEQ ID. No. 1, the partial sequence including an amino acid sequence from position-43 to position-67, from position-57 to position-81, from position-53 to position-68, from position-49 to position-64, from position-50 to position-60, or from position-51 to position-59, each of SEQ ID. No. 1.

9. A composition for inducing cellular immunity against lung cancer, the composition comprising a peptide comprising an amino acid sequence of any one of the following (f) to (k):
(f) an amino acid sequence from position-1 to position-39 of SEQ ID. No. 1;
(g) an amino acid sequence being a partial sequence of the amino acid sequence from position-1 to position-39 of SEQ ID. No. 1, the partial sequence including an amino acid sequence from position-1 to position-25, from position-15 to position-39, from position-13 to position-36, from position-13 to position-28, from position-17 to position-32, from position-21 to position-36, from position-9 to position-24, or from position-21 to position-29, each of SEQ ID. No. 1;
(h) an amino acid sequence from position-29 to position-53 of SEQ ID. No. 1;
(i) an amino acid sequence being a partial sequence of the amino acid sequence from position-29 to position-53 of SEQ ID. No. 1, the partial sequence including an amino acid sequence from position-29 to position-48, from position-29 to position-44, or from position-33 to position-48, each of SEQ ID. No. 1;
(j) an amino acid sequence from position-43 to position-81 of SEQ ID. No. 1;
(k) an amino acid sequence being a partial sequence of the amino acid sequence from position-43 to position-81 of SEQ ID. No. 1, the partial sequence including an amino acid sequence from position-43 to position-67, from position-57 to position-81, from position-53 to position-68, from position-49 to position-64, from position-50 to position-60, or from position-51 to position-59, each of SEQ ID. No. 1.

10. The composition according to claim 9, wherein the lung cancer is non-small-cell lung cancer or lung adenocarcinoma.
